(19) Europäisches Patentamt
European Patent Office
Office européen des brevets

(11) Veröffentlichungsnummer: **0 005 141**
**A1**

(12) # EUROPÄISCHE PATENTANMELDUNG

(21) Anmeldenummer: **79100325.4**

(22) Anmeldetag: **05.02.79**

(51) Int. Cl.²: **C 07 D 335/06,** A 61 K 31/38

(30) Priorität: **16.02.78 LU 79077**

(43) Veröffentlichungstag der Anmeldung: **14.11.79 Patentblatt 79/23**

(84) Benannte Vertragsstaaten: **BE CH DE FR GB IT NL SE**

(71) Anmelder: **CIBA-GEIGY AG, Patentabteilung Postfach, CH-4002 Basel (CH)**

(72) Erfinder: **Ferrini, Pier Giorgio, Dr., Im Rehwechsel 22, CH-4102 Binningen (CH)**

(54) **Benzothiopyranderivate, ihre Herstellung und diese enthaltende pharmazeutische Präparate.**

(57) Neue Benzothiopyranderivate der allgemeinen Formel

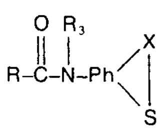

(I)

worin R gegebenenfalls verestertes oder amidiertes Carboxy oder gegebenenfalls veräthertes Hydroxymethyl bedeutet, Ph die Gruppe $R-CO-NR_3-$ enthaltendes, gegebenenfalls substituiertes 1,2-Phenylen bedeutet, X eine Gruppe der Formel $-CO-CR_1=CR_2-$ bedeutet, in der $R_1$ und $R_2$ unabhängig voneinander Wasserstoff, Acyl oder einen gegebenenfalls substituierten, gegebenenfalls heteroanalogen Kohlenwasserstoffrest oder gemeinsam 3- bis 5gliedriges Niederalkylen bedeuten und $R_2$ auch gegebenenfalls veräthertes oder mit einer organischen Carbonsäure verestertes Hydroxy sein kann, und $R_3$ Wasserstoff oder Niederalkyl bedeutet, besitzen antiallergische Eigenschaften und können als Arzneimittel verwendet werden. Sie werden z. B. hergestellt, indem man eine Verbindung der allgemeinen Formel

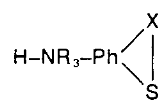

(II)

worin Ph, $R_3$ und X die angegebenen Bedeutungen haben, oder ein Salz davon mit einem Äquivalent einer gegebenenfalls funktionell abgewandelten Oxalsäure oder einem Salz davon umsetzt.

EP 0 005 141 A1

BEZEICHNUNG GEÄNDERT
siehe Titelseite

CIBA-GEIGY AG 4-11588/=
Basel (Schweiz)

Verfahren zur Herstellung neuer Benzothiopyranderivate

Gegenstand der Erfindung ist ein Verfahren zur Herstellung neuer Benzothiopyranderivate der allgemeinen Formel

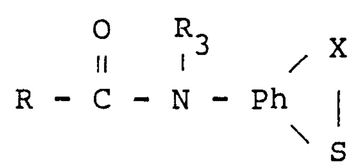

(I)

worin R gegebenenfalls verestertes oder amidiertes Carboxy bedeutet, Ph die Gruppe $R-CO-NR_3-$ enthaltendes, gegebenenfalls substituiertes 1,2-Phenylen bedeutet, X eine Gruppe der Formel $-CO-CR_1=CR_2-$ bedeutet, in der $R_1$ und $R_2$ unabhängig voneinander Wasserstoff, Acyl oder einen gegebenenfalls substituierten, gegebenenfalls heteroanalogen Kohlenwasserstoffrest oder gemeinsam 3- bis 5-gliedriges Niederalkylen bedeuten und $R_2$ auch gegebenenfalls veräthertes oder mit einer organischen Carbonsäure verestertes Hydroxy sein kann, und $R_3$ Wasserstoff oder Niederalkyl bedeutet, in freier Form oder in Salzform, die neuen Verbindungen selbst, diese enthaltende pharmazeutische Präparate und die Verwendung derselben.

Der Rest $R_3$ bedeutet vor allem Wasserstoff.

Verestertes Carboxy ist beispielsweise mit einem gegebenenfalls substituierten Alkohol aliphatischen oder aromatischen Charakters verestertes Carboxy.

Ein Alkohol aliphatischen Charakters ist ein Alkohol, dessen mit der Hydroxygruppe verbundenes C-Atom nicht Glied eines aromatischen Systems ist, beispielsweise ein gegebenenfalls durch gegebenenfalls substituiertes Aryl oder Heteroaryl, z.B. gegebenenfalls substituiertes Phenyl oder Pyridyl, substituierter aliphatischer Alkohol, als welcher z.B. ein Niederalkanol in Betracht kommt, oder ein cycloaliphatischer Alkohol, z.B. ein 5- bis 8-gliedriges Cycloalkanol. Als Beispiele für mit einem gegebenenfalls substituierten Alkohol aliphatischen Charakters verestertes Carboxy seien genannt: Niederalkoxycarbonyl, z.B. Methoxy-, Aethoxy-, Propoxy-, Isopropoxy- und Butoxycarbonyl, im Phenylteil gegebenenfalls substituiertes Phenylniederalkoxy-, vor allem α- und β-Phenylniederalkoxycarbonyl, wobei als gegebenenfalls substituiertes Phenyl und als Niederalkoxy insbesondere die nachstehend genannten in Betracht kommen, z.B. Benzyloxy- und α- sowie β-Phenäthoxycarbonyl, und 5- bis 8-gliedriges Cycloalkoxycarbonyl, z.B. Cyclopentyloxy-, Cyclohexyloxy- und Cycloheptyloxycarbonyl.

Ein Alkohol aromatischen Charakters ist ein Alkohol, dessen mit der Hydroxygruppe verbundenes C-Atom Glied eines carbocyclischen oder heterocyclischen aromatischen Systems ist, beispielsweise ein im Phenylteil gegebenenfalls substituiertes Phenol oder ein durch Niederalkyl, wie Methyl, oder Niederalkoxy, wie Methoxy, substituiertes Hydroxypyridin. Als Beispiel für mit einem gegebenenfalls

substituierten Alkohol aromatischen Charakters verestertes Carboxy seien genannt: Phenoxy-, Tolyloxy-, Anisyloxy- und Chlorphenoxycarbonyl, sowie 2-, 3-, 4-Pyridyloxycarbonyl.

Amidiertes Carboxy weist als Aminogruppe beispielsweise eine freie oder eine durch mindestens einen gegebenenfalls substituierten, gegebenenfalls heteroanalogen Kohlenwasserstoffrest aliphatischen Charakters oder einen gegebenenfalls substituierten Arylrest substituierte Aminogruppe auf.

In einen gegebenenfalls substituierten, gegebenenfalls heteroanalogen Kohlenwasserstoffrest aliphatischen Charakters geht die freie Valenz von einem nichtaromatischen C-Atom aus. Ein solcher Rest ist beispielsweise Niederalkyl oder Niederalkenyl, das durch gegebenenfalls substituiertes Phenyl oder Naphthyl substituiert sein kann, oder z.B. 5- bis 8-gliedriges, Cycloalkyl, wie Cyclohexyl, oder gegebenenfalls niederalkyliertes, z.B. methyliertes, gegebenenfalls monooxa-, -aza- oder -thianaloges 4- bis 7-gliedriges Alkylen, beispielsweise Tetra- oder Pentamethylen oder 3-Oxa-, 3-Aza- oder 3-Thiapentamethylen. Als Beispiele für durch mindestens einen solchen Rest substituiertes Carbamyl seien genannt: Mono- oder Diniederalkylcarbamyl, wie N-Methyl-, N,N-Diäthylcarbamyl, im Phenylteil gegebenenfalls wie nachstehend angegebenen substituiertes Phenylniederalkylcarbamyl, wie N-Benzyl- oder N-(1- oder 2-Phenäthyl)-carbamyl oder Pyrrolidino-, Piperidino-, Morpholino-, Thiomorpholino-, Piperazino- oder 4-Niederalkyl-, z.B. 4-Methylpiperazinocarbonyl.

Ein gegebenenfalls substituierter Arylrest ist beispielsweise gegebenenfalls substituiertes Naphthyl oder gegebenenfalls wie nachstehend angegeben und/oder an zwei

benachbarten Ringatomen durch eine Gruppe -OX- der angegebenen Bedeutung substituiertes Phenyl. Durch einen solchen Rest substituierte Carbamylgruppen sind beispielsweise N-Phenyl-, N-Tolyl-, N-Anisyl-, N-Chlorphenyl- und N-Naphthylcarbamyl zu nennen.

Die Gruppe $R-CO-NR_3-$ enthaltendes 1,2-Phenylen Ph kann ausser dieser Gruppe noch mindestens einen, z.B. einen oder zwei, weitere Substituenten, als welche beispielsweise Niederalkyl, wie die nachstehend genannten, z.B. Methyl,Niederalkoxy, wie die nachstehenden, z.B. Methoxy, Halogene wie die nachstehenden, z.B. Chlor und Trifluormethyl in Betracht kommen, aufweisen.

Acyl ist beispielsweise von einer organischen Carbonsäure oder von der gegebenenfalls partiell veresterten oder amidierten Kohlensäure abgeleitetes Acyl.

Von einer Carbonsäure abgeleitetes Acyl ist beispielsweise Niederalkanoyl oder gegebenenfalls substituiertes Benzoyl, beispielsweise Acetyl, Propionyl, Butyryl oder Benzoyl.

Von der gegebenenfalls partiell veresterten oder amidierten Kohlensäure abgeleitetes Acyl ist beispielsweise gegebenenfalls verestertes oder amidiertes Carboxyl, wie freies oder wie vorstehend angegeben verestertes oder amidiertes Carboxy, z.B. Carboxy, Methoxy- oder Aethoxycarbonyl oder Carbamyl.

Gegebenenfalls veräthertes Hydroxy ist beispielsweise gegebenenfalls mit einem Niederalkanol oder einem gegebenenfalls substituierten Phenol veräthertes Hydroxy, d.h. Hydroxy, Niederalkoxy oder gegebenenfalls substituiertes Phenoxy, beispielsweise Hydroxy, Methoxy, Aethoxy oder Phenoxy.

Gegebenenfalls mit einer Carbonsäure verestertes Hydroxy ist beispielsweise gegebenenfalls mit einer Niederalkancarbonsäure oder einer gegebenenfalls substituierten Benzoesäure verestertes Hydroxy, d.h. Hydroxy, Niederalkanoyloxy oder gegebenenfalls substituiertes Benzoyloxy, insbesondere Acetoxy, Propionyloxy oder Benzoyloxy.

Ein gegebenenfalls substituierter, gegebenenfalls heteroanaloger Kohlenwasserstoffrest ist beispielsweise ein gegebenenfalls substituierter Kohlenwasserstoffrest aliphatischen Charakters oder ein gegebenenfalls substituierter, gegebenenfalls heteroanaloger aromatischer Kohlenwasserstoffrest.

3- bis 5-gliedriges Niederalkylen kann geradkettig oder verzweigt sein und ist beispielsweise Propylen-1,3, Butylen-1,4, Pentylen-1,5 oder 2- oder 3-Methylbutylen-1,4.

In einem gegebenenfalls substituierten Kohlenwasserstoffrest aliphatischen Charakters geht die freie Valenz von einem nichtaromatischen C-Atom aus. Ein solcher Rest ist beispielsweise ein gegebenenfalls durch gegebenenfalls substituiertes Phenyl substituierter aliphatischer Kohlenwasserstoffrest, z.B. Niederalkylrest, ein cycloaliphatischer Kohlenwasserstoffrest, wie Adamantyl oder monocyclisches 5- bis 8-gliedriges Cycloalkyl oder Cycloalkenyl, z.B. 1-Cycloalkenyl. Als Beispiele für solche Reste sind insbesondere zu nennen: Methyl, Aethyl, Isopropyl und Butyle, Benzyl und Methyl-, Methoxy- und Chlorbenzyle, Cyclopentyl, Cyclohexyl, 1-Cyclohexenyl, Cycloheptyl und 1-Cycloheptenyl.

Ein gegebenenfalls substituierter, gegebenenfalls heteroanaloger aromatischer Kohlenwasserstoffrest weist beispielsweise 5 oder 6 Ringglieder und bis zu 2 Heteroatome, wie Stickstoff-, Sauerstoff- oder Schwefelatome, auf und ist beispielsweise gegebenenfalls substituiertes Phenyl, wie eines der nachstehenden, oder ein 5- oder 6-gliedriger, ein Stickstoff-, Sauerstoff- oder Schwefelatom aufweisender Heteroarylrest, wie beispielsweise einer der nachstehenden. Beispiele sind vor allem gegebenenfalls durch Methyl, Methoxy oder Chlor substituiertes Phenyl oder Pyridyl.

Vor- und nachstehend gilt:

Gegebenenfalls substituiertes Phenyl und Naphthyl sowie Phenyl in gegebenenfalls substituiertem Benzoyl, Benzoyloxy und aromatischen Alkoholen ist beispielsweise gegebenenfalls ein- oder mehrfach, z.B. ein- oder zweifach, substituiertes Phenyl oder Naphthyl, wobei als Substituenten vor allem Niederalkyl, Niederalkoxy oder Halogene, z.B. die nachstehend genannten, Hydroxy sowie Trifluormethyl in Betracht kommen, wie Phenyl, Naphthyl, o-, m- oder p-Tolyl, o-, m- oder p-Anisyl, o-, m- oder p-Chlorphenyl oder 2,4-, 3,5- oder 2,6-Dichlorphenyl.

Gegebenenfalls substituiertes Heteroaryl und solches in heteroaromatischen Alkoholen weist vorzugsweise 5 oder 6 Ringglieder und als Heteroatom(e) bis zu zwei Stickstoff-, Sauerstoff- und/oder Schwefelatome auf und ist beispielsweise gegebenenfalls ein- oder mehrfach substituiertes Pyridyl, Thienyl oder Furyl, wobei als Substituenten Niederalkyl, Niederalkoxy und Halogene, vor allem jeweils die nachstehend genannten in Betracht kommen, wie Pyridyl-2, -3 oder -4, 6-Methylpyridyl-2, 6-Methoxypyridyl-2 oder

2- oder 3-Thienyl.

Niederalkyl enthält beispielsweise bis zu 7, vor allem bis zu 4, C-Atome und kann geradkettig oder verzweigt sowie in beliebiger Stellung gebunden sein, wie Methyl, Aethyl, Propyl oder n-Butyl oder ferner Isopropyl, sek.- oder iso-Butyl.

Niederalkoxy sowie solches in Niederalkoxycarbonyl weist beispielsweise bis zu 7, vor allem bis zu 4, C-Atome auf und kann geradkettig oder verzweigt sowie in beliebiger Stellung gebunden sein, wie Methoxy, Aethoxy, Propoxy, Isopropoxy, Butoxy oder Amyloxy.

Niederalkanoyl sowie solches in Niederalkanoyloxy enthält beispielsweise bis zu 7,vor allem bis zu 4, C-Atome und kann geradkettig oder verzweigt sein, wie Acetyl, Propionyl, Butyryl oder Isobutyryl.

Halogen ist beispielsweise Halogen bis und mit Atomnummer 35, wie Fluor, Chlor oder Brom.

Salze von Verbindungen der allgemeinen Formel (I), worin R, $R_1$ und/oder $R_2$ für Carboxy steht, sind Salze mit Basen, in erster Linie entsprechende pharmazeutisch verwendbare Salze, wie Alkalimetall- oder Erdalkalimetall-, z.B. Natrium-, Kalium-, Magnesium- oder Calciumsalze, ferner Ammoniumsalze mit Ammoniak oder Aminen, wie Niederalkyl- oder Hydroxyniederalkylaminen, z.B. Trimethylamin, Triäthylamin oder Di- oder Tri-(2-hydroxyäthyl)-amin.

Die neuen Verbindungen zeigen wertvolle pharmakologische Eigenschaften. Insbesondere weisen sie antiallergische Wirkungen auf, die man z.B. an der Ratte in Dosen von etwa

1 bis etwa 100 mg/kg bei oraler Verabreichung im passiven kutanen Anaphylacie-Test (PCA-Reaktion), der analog der von Goose und Blair, Immunology, Bd. 16, S. 749 (1969) beschriebenen Methode durchgeführt wird, nachweisen kann. Dabei wird die passive kutane Anaphylaxie nach dem von Ovary, Progr. Allergy, Bd.5, S.459 (1958), beschriebenen Verfahren erzeugt. Sie bewirken ferner eine Hemmung der immunologisch induzierten Histaminfreisetzung, z.B. aus Peritonealzellen von Nippostrongylus brasiliensis-infestierten Ratten in vitro, Dukor et al., 11. Symposium des Collegium internationale allergologicum, Heidelberg Mai 1976, sie sind auch in verschiedenen Bronchokonstriktionsmodellen hochaktiv, wie sich z.B. im Dosisbereich von etwa 1 bis etwa 3 mg/kg i.v. anhand der durch IgE-Antikörper ausgelösten Bronchokonstriktion der Ratte und im Dosenbereich ab etwa 1 mg/kg i.v. anhand der durch IgG-Antikörper induzierten Bronchokonstriktion des Meerschweinchens zeigen lässt. Die Verbindungen der vorliegenden Erfindung sind deshalb als Hemmer von allergischen Reaktionen, z.B. in der Behandlung und Prophylaxe von allergischen Erkrankungen, wie Asthma, sowohl extrinsic als auch intrinsic Asthma, oder anderen allergischen Erkrankungen, wie Heufieber, Konjuktivitis, Urticaria und Ekzeme verwendbar.

Die Erfindung betrifft in erster Linie Verbindungen der allgemeinen Formel I, worin R Carboxy, mit einem Alkohol aliphatischen oder aromatischen Charakters verestertes Carboxy oder gegebenenfalls durch mindestens einen gegebenenfalls substituierten oder heteroanalogen Kohlenwasserstoffrest aliphatischen Charakters oder gegebenenfalls substituierten Arylrest substituiertes Carbamyl bedeutet, Ph die Gruppe $R-CO-NR_3-$ enthaltendes, gegebenenfalls substituiertes 1,2-Phenylen bedeutet, X eine Gruppe $-CO-CR_1=CR_2-$ bedeutet, in der $R_1$ und $R_2$ unabhängig voneinander Wasser-

stoff, Niederalkanoyl, Benzoyl gegebenenfalls wie vorstehend für R angegeben verestertes oder amidiertes Carboxy oder einen gegebenenfalls substituierten Kohlenwasserstoffrest aliphatischen Charakters oder gegebenenfalls heteroanalogen aromatischen Kohlenwasserstoffrest oder gemeinsam 1,3-, 1,4- oder 1,5-Niederalkylen bedeuten und $R_2$ auch gegebenenfalls mit einem Niederalkanol veräthertes oder mit einer Niederalkancarbonsäure verestertes Hydroxy bedeuten kann, und $R_3$ Wasserstoff oder Niederalkyl bedeutet, wobei als Substituenten von aromatischen und heteroaromatischen Gruppen jeweils vor allem Niederalkyl, wie Methyl, Niederalkoxy wie Methoxy, Halogen, wie Chlor, Hydroxy und Trifluormethyl in Betracht kommen, in freier Form oder in Salzform.

Die Erfindung betrifft vor allem Verbindungen der allgemeinen Formel I, worin R Carboxy, mit einem gegebenenfalls durch gegebenenfalls substituiertes Phenyl substituierten Niederalkanol oder einem gegebenenfalls substituierten Phenol verestertes Carboxy oder gegebenenfalls durch Niederalkyl, gegebenenfalls substituiertes Phenylniederalkyl oder gegebenenfalls substituiertes Phenyl mono- oder durch Niederalkyl oder gegebenenfalls heteroanaloges Niederalkylen disubstituiertes Carbamyl bedeutet, Ph die Gruppe R-CO-$NR_3$- enthaltendes, gegebenenfalls substituiertes 1,2-Phenylen bedeutet, X eine Gruppe -CO-$CR_1$=$CR_2$- bedeutet, in der $R_1$ und $R_2$ unabhängig voneinander Wasserstoff, Niederalkanoyl, wie Acetyl, gegebenenfalls mit einem Niederalkanol, wie Methanol, verestertes Carboxy, gegebenenfalls durch Phenyl, das seinerseits substituiert sein kann, substituiertes Niederalkyl oder gegebenenfalls substituiertes Phenyl oder 5- bis 6-gliedriges, ein Stickstoff-, Sauerstoff- oder Schwefelatom aufweisendes Heteroaryl oder gemeinsam Tri-, Tetra- oder Pentamethylen darstellen und

$R_2$ auch gegebenenfalls mit einem Niederalkanol, wie Methanol, veräthertes oder mit einer Niederalkancarbonsäure, wie Essigsäure, verestertes Hydroxy bedeuten kann, und $R_3$ Wasserstoff oder Niederalkyl bedeutet, wobei als Substituenten von Phenyl, Phenol, 1,2-Phenylen Ph und Heteroaryl, Niederalkyl, wie Methyl, Niederalkoxy, wie Methoxy, Halogen, wie Chlor, Hydroxy und Trifluormethyl in Betracht kommen, in freier Form oder in Salzform.

Die Erfindung betrifft insbesondere einerseits Verbindungen der allgemeinen Formel Ia

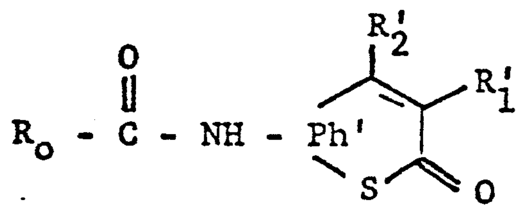

(Ia)

andererseits Verbindungen der allgemeinen Formel Ib

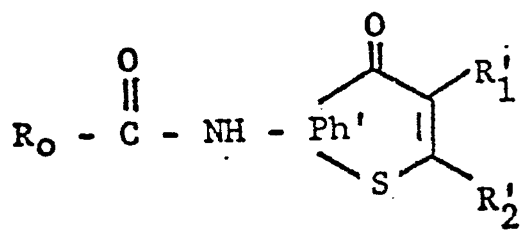

(Ib)

worin jeweils $R_o$ Carboxy, Niederalkoxycarbonyl, wie Methoxy- oder Aethoxycarbonyl, im Phenylteil gegebenenfalls wie nachstehend angegeben substituiertes Phenylniederalkoxycarbonyl, wie Benzyloxycarbonyl, oder gegebenenfalls durch Niederalkyl, wie Methyl oder Aethyl, mono- oder disubstituiertes oder durch Tetra- oder Pentamethylen bzw. 3-Oxa-, 3-Aza- oder 3-Thiapentamethylen disubstituiertes Carbamyl bedeutet, Ph' die Gruppe $R_o$-CO-NH- enthaltendes, zusätzliches gegebenenfalls wie nachstehend angegeben substituiertes 1,2-Phenylen bedeutet, $R_1'$ und $R_2'$ gemeinsam Tri-, Tetra- oder Pentamethylen bedeuten oder $R_1'$ Wasserstoff, Niederalkanoyl, wie Acetyl, Carboxy, Niederalkoxycarbonyl, wie Methoxy- oder Aethoxycarbonyl, Niederalkyl, wie Methyl,

oder gegebenenfalls wie nachstehend angegeben substituiertes Phenyl oder Pyridyl bedeutet und $R_2'$ eine der für $R_1'$ angegebenen Bedeutungen hat oder Hydroxy, Niederalkoxy, wie Methoxy, oder Niederalkanoyloxy, wie Acetoxy, bedeutet, wobei als Substituenten von substituiertem Phenylniederalkoxycarbonyl R', zusätzlich substituiertem 1,2-Phenylen Ph' und von substituiertem Phenyl und Pyridyl $R_1'$ und/oder $R_2'$ Niederalkyl, wie Methyl, Niederalkoxy, wie Methoxy, Halogen, wie Chlor, Hydroxy und Trifluormethyl in Betracht kommen, jeweils in freier Form oder in Salzform.

Die Erfindung betrifft in allererster Linie Verbindungen der allgemeinen Formel Ia, worin $R_o$ Carboxy oder Niederalkoxycarbonyl mit bis zu 5 C-Atomen, wie Methoxy- oder Aethoxycarbonyl, bedeutet, Ph' die, beispielsweise in 4- oder 5-Stellung gebundenen, Gruppe $R_o$-CO-NH enthaltendes, in einer der freien Stellungen gegebenenfalls durch Niederalkyl oder Niederalkoxy mit jeweils bis zu 4 C-Atomen, wie Methyl oder Methoxy, Hydroxy oder Halogen, wie Chlor, substituiertes 1,2-Phenylen bedeutet, und $R_1'$ und $R_2'$ unabhängig voneinander Wasserstoff, Niederalkyl mit bis zu 4 C-Atomen, wie Methyl, oder Phenyl bedeuten, jeweils in freier Form oder in Salzform.

Die Erfindung betrifft ganz besonders Verbindungen der Formel

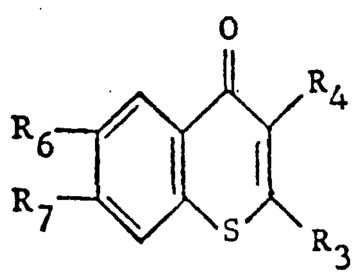

(Ic),

worin einer der Reste $R_6$ und $R_7$ eine Gruppe der Formel $R_o'$ -CO-NH, in der $R_o'$ Carboxy oder in zweiter Linie Niederalkoxycarbonyl mit bis zu 5 C-Atomen, wie Methoxy- oder

Aethoxycarbonyl, darstellt und der andere Wasserstoff darstellt, und $R_3$ und $R_4$ unabhängig voneinander Wasserstoff oder Niederalkyl mit bis zu 4 C-Atomen, wie Methyl, bedeuten, in freier Form oder in Salzform.

Die neuen Verbindungen können nach an sich bekannten Verfahren hergestellt werden.

Eine bevorzugte Arbeitsweise ist beispielsweise dadurch gekennzeichnet, dass man eine Verbindung der allgemeinen Formel II

```
              / X
H - NR3 - Ph  |          (II),
              \ S
```

worin Ph, $R_3$ und X die angegebenen Bedeutungen haben, oder ein Salz davon mit einem Aequivalent einer gegebenenfalls funktionell abgewandelten Oxalsäure oder einem Salz davon umsetzt und gewünschtenfalls eine so erhaltene Verbindung in eine andere Verbindung der allgemeinen Formel (I) umwandelt und/oder eine erhaltene salzbildende Verbindung in ein Salz oder ein erhaltenes Salz in die freie Verbindung überführt.

Salze von Verbindungen der Formel II sind beispielsweise Hydrohalogenide, wie Hydrochloride, derselben, ferner Salze mit Oxalsäure oder einem Monoester bzw. Monoamid davon.

Funktionelle Derivate der Oxalsäure sind mono- oder vorzugsweise difunktionelle Derivate der Oxalsäure, z.B. solche der Formel R-Y, worin Y eine gegebenenfalls veresterte, amidierte, oder, z.B. mit einer Halogenwasserstoffsäure, anhydridisierte Carboxylgruppe oder eine Gruppe der Formel $-C(=O)-N_3$ oder $-C(=O)-N_2^{\oplus} A^{\ominus}$, wobei $A^{\ominus}$ das

Anion einer Mineralsäure, z.B. Chlorid, Bromid oder Tetrafluoroborat ist, bedeutet. Als Beispiele seien insbesondere genannt: Symmetrische Oxalsäurediester, wie Diniederalkylester, und Diamide der Formel R-R sowie gegebenenfalls veresterte oder amidierte Halogenoxalsäuren der Formel R-CO-Hal, worin Hal Chlor oder Brom ist.

Die Umsetzung kann in üblicher, insbesondere in der aus der Literatur für analoge Umsetzungen bekannten, Weise erfolgen, erforderlichenfalls in Gegenwart eines Kondensationsmittel, bei der Umsetzung mit einem Esterhalogenid oder Amidhalogenid der Oxalsäure beispielsweise eines basischen Kondensationsmittels, wie einer tertiären organischen Stickstoffbase, z.B. von Triäthylamin oder Pyridin, oder eines Alkalimetallhydroxides oder -carbonats, z.B. von Natrium- oder Kaliumhydroxid, oder bei der Umsetzung mit Oxalsäure beispielsweise eines die Dehydratisierung des primär gebildeten Ammoniumsalzes bewirkenden Kondensationsmittels, wie eines wasserbindenden Mittels, z.B. von Dicyclohexylcarbodiimid oder eines Isonitrils, wie tert.-Butylisonitril, oder einer Mineralsäure, z.B. von Chlorwasserstoffsäure, oder eines Säureanhydrides, z.B. von Phosphorpentoxid, und/oder in einem inerten Lösungsmittel, vorzugsweise einem inerten polaren Lösungsmittel, wie einem N,N-Dialkylamid, z.B. in N,N-Dimethylformamid oder -acetamid.

Die neuen Verbindungen können ferner hergestellt werden, indem man in einer Verbindung der Formel

```
          /X
R' - Ph  |            (III)
          \S
```

worin R' einen in die gewünschte Gruppe der Formel $RCO-NR_3$-überführbaren Rest bedeutet, R' in die Gruppe der Formel

RCO-$NR_3$- überführt und gewünschtenfalls eine so erhältliche Verbindung in eine andere Verbindung der Formel I umwandelt und/oder ein erhaltenes Salz in die freie Verbindung oder in ein anderes Salz oder eine erhaltene salzbildende Verbindung in ein Salz überführt.

Ein in die Gruppe der Formel RCO-$NR_3$- überführbarer Rest ist beispielsweise ein durch Solvolyse, d.h. Hydrolyse, Alkoholyse (Umsetzung mit dem der gewünschten veresterten Carboxygruppe R- entsprechenden Alkohol) und/oder Aminolyse (Umsetzung mit Ammoniak oder einem der gewünschten amidierten Carboxygruppe entsprechenden Amin) in diese überführbarer Rest, vorzugsweise eine Gruppe der Formel $X_1$-$NR_3$-, in der $X_1$ eine von einer gegebenenfalls veresterten oder amidierten Oxalogruppe verschiedene und in diese überführbare funktionell abgewandelte Oxalogruppe bedeutet. Derartige funktionell abgewandelte Oxalogruppen sind vorzugsweise solche, die als funktionell abgewandelte α-Carbonylgruppierung Thioxomethylen, Iminomethylen oder eine veresterte und/oder verätherte Dihydroxymethylengruppierung und/oder als funktionell abgewandelte Carboxygruppe eine von einer veresterten oder amidierten Carboxygruppe verschiedene funktionell abgewandelte Carboxygruppe aufweisen. Veresterte und/oder verätherte Dihydroxymethylengruppierungen sind beispielsweise mit einer Halogenwasserstoffsäure, wie Chlorwasserstoffsäure, veresterte und/oder mit einem Niederalkanol, wie Methanol oder Aethanol verätherte Dihydroxymethylengruppen. Als Beispiele seien vor allem Dihalogenmethylengruppierungen, wie Dichlormethylen, Niederalkoxyhalogenmethylengruppierungen, wie Methoxy- oder Aethoxychlormethylen oder Diniederalkoxymethylengruppierungen, wie Dimethoxy- oder Diäthoxymethylen, genannt. Von veresterten oder amidierten Carboxylgruppen verschiedene funktionell abgewandelte Carboxygruppierungen sind beispielsweise die Cyanogruppe, anhydridisierte Carboxygruppen, wie Halogen-, z.B. Chlorcarbonyl, Iminoester-, wie Imid- bzw. Amidhalogenidgruppierungen, z.B. Iminochlor- oder Aminodichlormethyl, Iminoäthergruppierungen, wie Niederalkyl- oder Niederalkyleniminoäthergruppierungen, z.B. Methoxy- oder Aethoxyiminomethylen, 4,4- oder 5,5-Dimethyloxazolinyl-(2) oder 4,4,6-Trimethyl-dihydro-oxazinyl-(2), Amidinogruppen, wie Amidino oder Niederalkyl-, z.B. Methylamidino, mit einer Halogenwasserstoffsäure, wie Chlorwasserstoffsäure, veresterte und/oder mit einem Niederalkanol verätherte Orthosäuregruppierungen, wie Triniederalkoxy-, Niederalkoxyhalogen- oder Trihalogenmethylgruppen, vor allem Trimethoxy- oder Triäthoxymethyl, Aethoxydichlormethyl oder Trichlormethyl, oder gegebenenfalls veresterte Thiocarboxylgruppen, wie Niederalkylthiocarbonylgruppen, z.B. Aethylthiocarbonyl. Derartige Gruppen $X_1$ können z.B. hydrolytisch in die Oxalogruppe, überführt werden. Als funktionell abgewandelte Carboxygruppe eine Iminoäther-, Orthoester- oder Esterhalogenidgruppierung und/oder als funktionell abgewandelte α-Carbonylgruppe Thioxo- oder Iminomethylen oder eine veresterte oder verätherte Dihydroxymethylengruppe aufweisende Gruppen $X_1$ können ferner zu veresterten Oxalogruppen hydrolysiert werden. Ebenso können als funktionell abgewandelte Carboxygruppe die Cyanogruppe, eine Amidino- oder Imid- bzw. Amidhalogenidgruppierung und/oder als funktionell abgewandelte α-Carbonylgruppe Thioxo- oder Iminomethylen oder eine verätherte oder veresterte Dihydroxymethylengruppe aufweisende Gruppen $X_1$ zu amidierten Carboxygruppen hydrolysiert werden. Die Hydrolyse kann in üblicher Weise durchgeführt werden, erforderlichenfalls in Gegenwart eines basischen oder vorzugsweise sauren Hydrolysemittels, wie eines Alkalimetallhydroxydes, wie Natrium- oder Kaliumhydroxyd, oder vorzugsweise einer Protonensäure, vorzugsweise einer Mineralsäure, z.B. einer Halogenwasserstoffsäure, wie Salzsäure oder einer organischen Carbon- oder Sulfonsäure, z.B. von Essigsäure oder p-Toluolsulfonsäure.

So kann man beispielsweise eine beliebige, von einer veresterten oder amidierten Oxaloaminogruppe RCO-verschiedenen funktionell abgewandelte Oxalogruppe $X_1$ durch Hydrolyse, beispielsweise in Gegenwart eines sauren oder basischen Mittels, wie einer Mineralsäure, z.B. von Salzsäure, oder eines Alkalimetallhydroxides, z.B. von Natron- oder Kalilauge, bei der Hydrolyse von Cyanocarbonyl- oder von Thiooxalogruppen zur Oxalogruppe vorzugsweise sauer und/oder in Gegenwart eines Oxydationsmittels, z.B. von Wasserstoffperoxid, in die freie Oxalogruppe überführen. Dabei arbeitet man erforderlichenfalls in einem polaren Lösungsmittel, wie einem Niederalkanol, Keton oder Aether, z.B. in Aethanol, Aceton oder Dioxan, und/oder unter Kühlen oder Erwärmen, z.B. bei etwa 0°C bis etwa 100°C.

Als funktionell abgewandelte Carboxygruppe eine anhydridisierte Carboxygruppe, wie Halogencarbonyl, z.B. Chlorcarbonyl, Cyanocarbonyl oder eine Niederalkyleniminoäthergruppierung, z.B. 4,4- oder 5,5-Dimethyl-oxazolinyl-(2), oder 4,4,6-Trimethyl-dihydro-oxazinyl-(2), aufweisende funktionell abgewandelte Oxalogruppen können ferner durch übliche Alkoholyse, d.h. Umsetzung mit dem entsprechenden Alkohol, in veresterte Oxalogruppen überführt werden. Bei der Alkoholyse von anhydridisierten Carboxygruppen arbeitet man vorteilhaft in Gegenwart eines basischen Kondensationsmittels, z.B. von Pyridin oder Triäthylamin, während man die Alkoholyse einer Niederalkyleniminoäthergruppierung vorzugsweise sauer, z.B. in Gegenwart von Chlorwasserstoffäure, p-Toluolsulfonsäure oder Essigsäure, durchführt. In analoger Weise kann man eine anhydridisierte

Carboxygruppe aufweisende funktionell abgewandelte Oxalogruppe auch durch Ammono- bzw. Aminolyse, d.h. Umsetzung mit Ammoniak oder einem entsprechenden primären oder sekundären Amin, vorzugsweise in Gegenwart eines basischen Kondensationsmittels, z.B. von Natriumhydroxid, Pyridin oder Triäthylamin, in eine amidierte Oxalogruppe R-C(=O)- überführen.

Weitere in Gruppen der Formel RCO-$NR_3$- überführbare Reste R' sind beispielsweise oxydativ in diese überführbare Gruppen, insbesondere solche der Formel $X_2$-$NR_3$-, worin $X_2$ die oxydativ in die Oxalogruppe der Formel R-C(=O)-, worin R Carboxy darstellt, überführbare gegebenenfalls hydratisierte Glyoxylgruppe bedeutet. Diese kann vorteilhaft im Verlaufe der Oxydationsreaktion, z.B. aus der Acylgruppe einer gegebenenfalls α,β-ungesättigten oder α,β-dihydroxylierten aliphatischen oder araliphatischen Carbonsäure, einer gegebenenfalls an der Hydroxygruppe veresterten Glykoloylgruppe oder der Glycylgruppe, in situ gebildet oder aus einem ihrer funktionellen Derivate, z.B. einem ihrer Acetale oder Imine in Freiheit gesetzt werden. Acylgruppen von gegebenenfalls α,β-ungesättigten oder α,β-dihydroxylierten Carbonsäuren sind beispielsweise Alkanoylgruppen, wie Niederalkanoyl, z.B. Acetyl, Acylgruppen von α,β-ungesättigten aliphatischen Mono- oder Dicarbonsäuren, z.B. Acryloyl, Crotonyl oder die Acylgruppe der gegebenenfalls funktionell abgewandelten Fumar- oder Maleinsäure, Acylgruppen von α,β-ungesättigten araliphatischen Carbonsäuren, z.B. gegebenenfalls substituiertes Cinnamoyl, oder Acylgruppen von aliphatischen α,β-Dihydroxydicarbonsäuren, wie der Weinsäure, oder monofunktioneller Carboxyderivate, wie Estern oder Amiden, derselben. Veresterte Glykoloylgruppen sind beispielsweise an der Hydroxygruppe mit einer Mineralsäure, wie einer Halogenwasserstoffsäure, z.B. mit

Chlor- oder Bromwasserstoffsäure, oder mit einer Carbonsäure, z.B. mit Essigsäure oder der gegebenenfalls substituierten Benzoesäure, veresterte Glykoloylgruppen. Acetalisierte Glyoxyloylgruppen sind beispielsweise mit Niederalkanolen oder einem Niederalkandiol acetalisierte Glyoxyloylgruppen, wie Dimethoxy-, Diäthoxy- oder Aethylendioxyacetyl. Imine von Glyoxyloylgruppen sind beispielsweise gegebenenfalls substituierte N-Benzylimine oder N-(2-Benzothiazolyl)-imine derselben oder Imine mit 3,4-Di-tert.-butyl-o-chinon. Weitere oxydativ in die Oxalogruppe überführbare Reste sind z.B. gegebenenfalls substituierte, wie in 5-Stellung eine acetalisierte Formylgruppe, wie Diäthoxymethyl, aufweisende 2-Furoylgruppen. Zu veresterten Oxaloaminogruppen der Formel R-C(=O)-, worin R für verestertes Carboxy steht, oxydierbare Gruppen sind verätherte Glykoloylgruppen, wie Niederalkoxyacetyl. Zu gegebenenfalls veresterte oder amidierte Oxaloaminogruppen oxydierbare Reste sind ferner gegebenenfalls hydratisierte oder acetalisierte Formylmethylaminogruppen oder gegebenenfalls funktionell abgewandelte Carboxymethylaminogruppen bzw. Carboxymethyleniminogruppen, z.B. der Formel $OHC-CH_2-NR_3-$, $R-CH_2-NR_3-$ bzw. R-CH=N-.

Die Oxydation kann in üblicher Weise durch Umsetzung mit einem geeigneten Oxydationsmittel erfolgen. Geeignete Oxydationsmittel sind insbesondere oxydierende Schwermetallverbindungen, wie Silberverbindungen, z.B. Silbernitrat oder Silberpicolinat, Sauerstoffsäuren von Schwermetallen, z.B. von Mangan-IV, Mangan-VII, Chrom-VI und Eisen-VI, oder von Halogenen bzw. deren Anhydride oder Salze, wie Chromsäure, Chromdioxid, Kaliumdichromat, Kaliumpermanganat, Mangandioxid, Kaliumferrat, Natriumchlorit in Gegenwart von Sulfaminsäure, Natriumhypochlorit in Gegenwart von Nickelchlorid oder Natriumjodat, Natriumper-

jodat oder Bleitetraacetat. Die Umsetzung mit diesen Oxydationsmitteln erfolgt in üblicher Weise, beispielsweise in einem inerten Lösungsmittel, wie Aceton, Essigsäure, Pyridin oder Wasser, oder einem, vorzugsweise wässrigen, inerten Lösungsmittelgemisch, bei Normaltemperatur oder erforderlichenfalls unter Kühlen oder Erwärmen, z.B. bei etwa 0°C bis etwa 100 °C. Die Oxydation von gegebenenfalls verätherten Glykoloylgruppen zu gegebenenfalls veresterten Oxalogruppen wird z.B. vorteilhaft mit Kaliumpermanganat in wässrigem Pyridin oder Aceton bei Raumtemperatur vorgenommen. Acetalisierte Glyoxyloylgruppen und Iminoacetylgruppen werden vorzugsweise sauer oxydiert, z.B. mit Kaliumdichromat in Schwefelsäure, Acylgruppen von α,β-dihydroxylierten aliphatischen Carbonsäuren, wie der Acylrest der Weinsäure werden vorteilhaft mit Perjodsäure oxydiert, während man für die Oxydation der Glycylgruppe vorzugsweise Kaliumferrat in alkalischem Milieu, z.B. bei $p_H$ = 10-13, z.B. 11.5, oder organische Silbersalze, wie Silberpicolinat verwendet. Gruppen der Formel R-CH=N- werden vorzugsweise mit einer organischen Persäure, z.B. mit Peressigsäure oder m-Chlorperbenzoesäure, in einem inerten Lösungsmittel, z.B. Methylenchlorid, Chloroform oder Benzol, oxydiert.

Eine andere Arbeitsweise besteht darin, dass man eine Verbindung der allgemeinen Formel

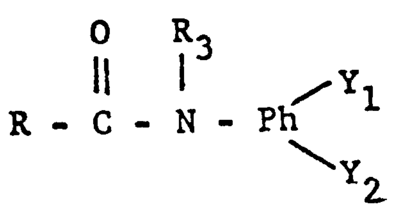

(IV) ,

worin entweder $Y_1$ eine Gruppe $-CR_2=CR_1-Y_3$ bzw. $-CO-CR_1=CR_2-Y_4$ und $Y_2$ gegebenenfalls veräthertes oder mit einer Carbonsäure verestertes Mercapto bedeutet, oder $Y_1$ Wasserstoff und $Y_2$ eine Gruppe $-S-CR_2=CR_1-Y_3$ bzw. $-S-CO-CR_1=CR_2-Y_4$ bedeutet, oder $Y_1$ eine Gruppe $-C(C)Y_5$ und $Y_2$ eine

Gruppe $-S-C(O)-Y_6$ bedeutet, und worin $Y_3$ gegebenenfalls funktionell abgewandeltes Carboxy bedeutet, $Y_4$ gegebenenfalls veräthertes oder verestertes Mercapto bedeutet, wobei eine Gruppe $-CO-CR_1=CR_2-OH$ bzw. $-S-CO-CR_1=CR_2-OH$ auch in der durch die Formel $-CO-CHR_1-C(O)-R_2$ bzw. $-S-CO-CHR_1-C(O)-R_2$ dargestellten tautomeren Oxoform vorliegen kann, einer der Reste $Y_5$ und $Y_6$ eine Gruppe $-CH_2R_1$ und der andere eine Gruppe $R_2$ oder veräthertes Mercapto bedeutet, und R, Ph, $R_1$, $R_2$ und $R_3$ die angegebenen Bedeutungen haben, oder ein Salz davon intramolekular cyclisiert und in einer gegebenenfalls erhaltenen Verbindung der Formel

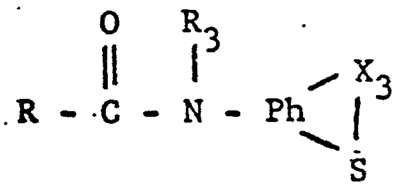

(I')

worin $X_3$ eine Gruppe $-C(=Y_o)-CR_1=CR_2-$ und $Y_o$ eine funktionell abgewandelte Oxogruppe bedeutet, $Y_o$ in die Oxogruppe überführt, erforderlichenfalls ein erhaltenes Isomerengemisch in die Komponenten auftrennt und gewünschtenfalls eine oder mehrere der genannten fakultativen Zusatzoperationen durchführt.

Funktionell abgewandeltes Carboxy ist beispielsweise Cyano oder verestertes oder anhydridisiertes Carboxy. Verestertes Carboxy ist vorzugsweise mit einem Niederalkanol verestertes Carboxy, z.B. Methoxycarbonyl oder Aethoxycarbonyl. Anhydridisiertes Carboxy ist beispielsweise mit einer Carbonsäure, wie mit einer Niederalkancarbonsäure oder einer gegebenenfalls substituierten Benzoesäure, oder mit einer Halogenwasserstoffsäure anhydridisiertes Carboxy, z.B. Acetoxycarbonyl, Benzoyloxycarbonyl oder Brom- bzw. Chlorcarbonyl.

Eine funktionell abgewandelte Oxogruppe $Y_o$ ist

beispielsweise eine Thioxo- oder vor allem Iminogruppe, vorzugsweise die primäre Iminogruppe, welche auch in Form eines Säureadditionssalzes,z.B. des Hydrochlorides, vorliegen kann.

Veräthertes Mercapto ist beispielsweise Niederalkylthio, z.B. Methylthio oder Aethylthio.

Verestertes Mercapto ist beispielsweise mit einer Carbonsäure verestertes Mercapto.

Mit einer Carbonsäure verestertes Mercapto ist beispielsweise Niederalkanoylthio oder gegebenenfalls substituiertes Benzoylthio, z.B. Acetylthio oder Benzoylthio.

Salze von Verbindungen der allgemeinen Formel (IV) sind insbesondere Metallsalze, wie Alkalimetallsalze, z.B. Natrium- oder Kaliumsalze, von Verbindungen mit einer phenolischen Mercaptogruppe $Y_2$, Carboxylgruppe $Y_3$ und/oder R, enolischen Hydroxygruppe $Y_4$ und/oder $R_1CH_2$-Gruppen $Y_5$ oder $Y_6$.

Die intramolekulare Cyclisierung kann in üblicher Weise, insbesondere in der aus der Literatur für analoge Reaktionen bekannten, Weise erfolgen, erforderlichenfalls in Gegenwart eines Kondensationsmittels und/oder bei erhöhter Temperatur, z.B. der Siedetemperatur der Reaktionsmischung.

Kondensationsmittel sind dabei beispielsweise die üblichen sauren oder basischen Kondensationsmittel. Saure Kondensationsmittel sind beispielsweise Protonensäuren, wie Carbonsäuren, z.B. Essig- oder Trifluoressigsäure, organische Sulfonsäuren, z.B. Benzol-, p-Toluol-, p-Brombenzol-oder Methansulfonsäure, oder vor allem Mineralsäuren und gegebenenfalls ihre sauren Salze, wie Chlor-, Brom- oder Jodwasserstoffsäure, Schwefelsäure, Kaliumhydrogensulfat, Phosphorsäure oder Polyphosphorsäure, oder Säureanhydride, z.B. Acetanhydrid, Acetylchlorid, Phosphorpentoxid oder Phosphoroxychlorid, oder Lewissäuren, vorzugsweise Halogenide, z.B. Fluoride, Chloride oder Bromide von Elementen der 3., 4. oder 5. Hauptgruppe oder 2. Nebengruppe des periodischen Systems, wie des Bors, Aluminiums, Antimons, Zinks, Zinns oder Cadmiums, z.B. Aluminiumtrichlorid oder -bromid, Brotrifluorid, Zinkchlorid oder Antimonpentachlorid. Basische Kondensationsmittel sind beispielsweise schwach oder mässig basische Kondensationsmittel, wie Alkalimetall- oder Ammoniumsalze von Carbonsäuren, z.B. Natriumacetat, oder tertiäre organische Stickstoffbasen, z.B. Pyridin oder Triäthylamin, oder stark basische Kondensationsmittel, wie Alkalimetalle oder deren Hydride, Amide, Alkoholate oder Kohlenwasserstoffverbindungen, z.B. Natrium, Kalium, Natriumhydrid, Natriumamid, Diisopropylaminlithium, Natriummethylat, Natriumäthylat oder Tritylnatrium.

Inerte Lösungsmittel sind beispielsweise polare inerte Lösungsmittel, wie ein Ueberschuss der als Kondensationsmittel genannten Säuren, Säureanhydride oder tertiären Stickstoffbasen, Alkohole, z.B. Methanol, Aethanol, Glykol, Glykol- oder Diäthylenglykolmonomethyläther, Ketone, z.B. Aceton, N,N-Dialkylcarbonsäureamide, z.B. Dimethylformamid, Sulfoxide, z.B. Dimethylsulfoxid, oder Carbonsäuren, wie Niederalkansäuren oder deren Anhydride, z.B. Essigsäure, Acetanhydrid oder Acetylchlorid.

Die Ueberführung eines gegebenenfalls erhaltenen Zwischenproduktes der Formel I' erfolgt beispielsweise durch milde Hydrolyse und kann gleichzeitig mit oder anschliessend an die Cyclisierung erfolgen, im letzteren Falle z.B. durch kurzfristige Einwirkung einer wässrigen Säurelösung, z.B. von Salzsäure.

So kann man in einer bevorzugten Ausführungsform des vorstehenden Verfahrens beispielsweise eine Verbindung der allgemeinen Formel IV, worin $Y_1$ eine Gruppe der Formel $-CR_2=CR_1-Y_3$, $Y_2$ Wasserstoff oder einen gegebenenfalls substituierten, gegebenenfalls heteroanalogen Kohlenwasserstoffrest, $R_2$ gegebenenfalls veräthertes oder mit einer Carbonsäure verestertes Mercapto und $Y_3$ gegebenenfalls verestertes oder anhydridisiertes Carboxy oder Cyano bedeutet, erforderlichenfalls in Gegenwart eines sauren Kondensationsmittels, zu Verbindungen der allgemeinen Formel I, worin -SX- eine Gruppe $-SOC-CR_1=CR_2-$ ist, umsetzen. Ausgehend von Ausgangsstoffen, in denen $Y_3$ gegebenenfalls verestertes Carboxy ist, verwendet man als Kondensationsmittel beispielsweise eine Phosphorsäureanhydrid, wie Phosphorpentoxid oder Phosphorpentachlorid oder eine Protonensäure, z.B. Jod-, Chlor oder Bromwasserstoffsäure, Schwefelsäure oder Trifluoressigsäure. Ausgehend von einem Ausgangsstoff in dem $Y_2$ veräthertes Mercapto und $Y_3$ Cyano ist, cyclisiert man vorzugsweise in Gegenwart einer Lewissäure, z.B. von Aluminiumtrichlorid, wobei das primär gebildete Zwischenprodukt der Formel I' in dem sauren Milieu der Aufarbeitung hydrolysiert wird.

In einer anderen bevorzugten Ausführungsform des vorstehenden Verfahrens kann man beispielsweise eine Verbindung der allgemeinen Formel IV, worin $Y_1$ eine Gruppe der Formel $-COCHR_1-CO-R_2$ und $Y_2$ gegebenenfalls veräthertes Mercapto oder $Y_1$ eine Gruppe der Formel $-SCR_2=CR_1-Y_3$ und $Y_2$ Wasserstoff darstellt, und worin $R_2$ Wasserstoff oder

einen gegebenenfalls substituierten, gegebenenfalls heteroanalogen Kohlenwasserstoffrest und $Y_3$ gegebenenfalls verestertes oder mit einer Halogen-, z.B. mit Chlorwasserstoffsäure, anhydridisiertes Carboxy bedeutet, in Gegenwart eines sauren Kondensationsmittels zu einer Verbindung der allgemeinen Formel I, worin -XS- eine Gruppe $-COCR_1-CR_2S-$ ist, cyclisieren. Als saures Kondensationsmittel wird dabei ausgehend von Ausgangsstoffen, in denen $Y_1$ eine Gruppe $-S-CR_2=CR_1-Y_3$ und $Y_3$ gegebenenfalls verestertes Carboxy ist, vorzugsweise eine Mineralsäure, z.B. Schwefelsäure oder Polyphosphorsäure, Jodwasserstoffsäure oder Bromwasserstoffsäure in Essigsäure, oder ein Säureanhydrid, z.B. Phosphorpentoxid oder Phosphorpentachlorid, oder ausgehend von Ausgangsstoffen, in denen $Y_1$ eine Gruppe $-S-CR_2=CR_1-Y_3$ und $Y_3$ Halogen-, wie Chlorcarbonyl ist, vorzugsweise eine Lewissäure, z.B. Aluminiumtrichlorid, verwendet.

In einer weiteren bevorzugten Ausführungsform des vorstehenden Verfahrens kann man beispielsweise eine Verbindung der allgemeinen Formel IV, worin $Y_1$ gegebenenfalls verestertes Carboxy und $Y_2$ eine Gruppe $-S-CO-CH_2R_1$ darstellt, und $R_2$ Wasserstoff oder einen gegebenenfalls substituierten, gegebenenfalls heteroanalogen Kohlenwasserstoffrest bedeutet, zu Verbindungen der allgemeinen Formel I, worin -SX- eine Gruppe $-SCOCR_1=C(OH)-$ bedeutet cyclisieren. Vorzugsweise arbeitet man in Gegenwart eines basischen Kondensationsmittels, vor allem eines stark basischen Kondensationsmittels, z.B. von Natrium, Natriumhydrid oder Natriumäthylat.

Eine erfindungsgemäss erhältliche Verbindung der allgemeinen Formel I kann in an sich bekannter Weise in eine andere Verbindung der allgemeinen Formel I umgewandelt werden.

So kann man beispielsweise eine freie Carboxylgruppe R in üblicher Weise, z.B. durch Behandeln mit einem gegebenenfalls durch gegebenenfalls substituiertes Aryl oder Heteroaryl substituierten Diazoniederalkan oder Triniederalkyloxonium- oder Triniederalkylcarboxoniumsalz, wie Hexachloroantimonat oder Hexafluorophosphat, oder vor allem durch Umsetzung mit dem entsprechenden Alkohol oder einem reaktionsfähigen Derivat, wie einem N,N-Diniederalkyl-alkansäureamid-acetal, z.B. einem N,N-Dimethyl-formamid-diniederalkylacetal, einem N,N,O-Triniederalkyl-formamidsalz, z.B. einem N,N,O-Triniederalkyl-formamid-metho-sulfat,oder einem Carbon-, Phosphorig-,Schweflig- oder Kohlensäureester, z.B. einem Niederalkancarbonsäureester, Triniederalkylphosphit, Diniederalkylsulfit, in Gegenwart eines schwach bis mässig basischen Kondensationsmittels, zu verestertem Carboxy verestern.

Die Umsetzung mit dem entsprechenden Alkohol selbst kann vorteilhaft in Gegenwart eines sauren Katalysators erfolgen, wie einer Protonensäure, z.B. von Chlor- oder Bromwasserstoff-, Schwefel-, Phosphor-, Bor-, Benzolsulfon- und/oder Toluolsulfonsäure, in einem inerten Lösungsmittel, insbesondere einem Ueberschuss des eingesetzten Alkohols und erforderlichenfalls in Gegenwart eines wasserbildenden Mittels und/oder unter destillativer, z.B. azetroper, Entfernung des Reaktionswassers und/oder bei erhöhter Temperatur.

Die Umsetzung mit einem reaktionsfähigen Derivat des entsprechenden Alkohols kann in üblicher Weise durchgeführt werden, ausgehend von einem Carbon-, Phosphorig-, Schweflig- oder Kohlensäureester beispielsweise in Gegenwart eines sauren Katalysators, wie eines der vorstehend genannten, in einem inerten Lösungsmittel, wie einem aromatischen Kohlenwasserstoff, z.B. in Benzol oder Toluol,

oder einem Ueberschuss des eingesetzten Alkoholderivates oder des entsprechenden Alkohols, erforderlichenfalls unter, z.B. azeotroper, Abdestillation des Reaktionswassers. Ausgehend von einem Mineralsäure- oder Sulfonsäureester setzt man die zu veresternde Säure vorteilhaft in Form eines Salzes, z.B. des Natrium- oder Kaliumsalzes, ein und arbeitet erforderlichenfalls in Gegenwart eines basischen Kondensationsmittels, wie einer anorganischen Base, z.B. von Natrium- oder Kalium- oder Calciumhydroxid oder -carbonat, oder einer tertiären organischen Stickstoffbase, z.B. von Triäthylamin oder Pyridin, und/oder in einem inerten Lösungsmittel, wie einer der vorstehenden tertiären Stickstoffbasen oder eines polaren Lösungsmittels, z.B. in Dimethylformamid und/oder bei erhöhter Temperatur.

Die Umsetzung mit einem Olefin kann beispielsweise in Gegenwart eines sauren Katalysators, z.B. einer Lewissäure, z.B. von Bortrifluorid, einer Sulfonsäure, z.B. von p-Toluolsulfonsäure, oder vor allem eines basischen Katalysators, z.B. von Natrium- oder Kaliumhydroxid, vorteilhaft in einem inerten Lösungmittel, wie einem Aether, z.B. in Diäthyläther oder Tetrahydrofuran, erfolgen.

Eine freie Carboxylgruppe R kann ferner durch Umsetzung mit Ammoniak oder einem mindestens ein Wasserstoffatom aufweisenden Amin in üblicher Weise, unter Dehydratisierung des intermediär gebildeten Ammoniumsalzes, z.B. durch azeotrope Destillation mit Benzol oder Toluol oder trockenes Erhitzen, in eine amidierte Carboxylgruppe R überführt werden.

Die vorstehend beschriebenen Umwandlungen freier in veresterte oder amidierte Carboxylgruppen R können aber auch so durchgeführt werden, dass man eine Verbindung der

Formel I, worin R Carboxyl ist, zunächst in üblicher Weise in ein reaktionsfähiges Derivat, beispielsweise mittels eines Halogenides des Phosphors oder Schwefels, z.B. mittels Phosphortrichlorid oder -bromid, Phosphorpentachlorid oder Thionylchlorid, in ein Säurehalogenid oder durch Umsetzung mit einem entsprechenden Alkohol oder Amin in einen reaktiven Ester, d.h. Ester mit elektronenanziehenden Strukturen, wie den Ester mit Phenol, Thiophenol, p-Nitrophenol oder Cyanmethylalkohol, oder ein reaktives Amid, z.B. das von Imidazol oder 3,5-Dimethylpyrazol abgeleitete Amid, überführt und das erhaltene reaktionsfähige Derivat dann in üblicher Weise, z.B. wie nachstehend für die Umesterung, Umamidierung bzw. gegenseitige Umwandlung veresterter und amidierter Carboxylgruppen R beschrieben, mit einem entsprechenden Alkohol, Ammoniak oder dem entsprechenden mindestens ein Wasserstoffatom aufweisenden Amin zu der gewünschten Gruppe R umsetzt.

Eine veresterte Carboxylgruppe R kann in üblicher Weise, z.B. durch Hydrolyse in Gegenwart eines Katalysators, beispielsweise eines basischen oder sauren Mittels, wie einer starken Base, z.B. von Natrium- oder Kaliumhydroxid, oder einer Mineralsäure, z.B. von Salzsäure, Schwefelsäure oder Phosphorsäure, zur freien Carboxylgruppe R oder z.B. durch Umsetzung mit Ammoniak oder dem entsprechenden, mindestens ein Wasserstoffatom aufweisenden Amin in eine amidierte Carboxylgruppe R überführt werden.

Eine veresterte Carboxylgruppe R kann ferner in üblicher Weise, z.B. durch Umsetzung mit einem Metallsalz, wie dem Natrium- oder Kaliumsalz, eines entsprechenden Alkohols oder mit diesem selbst in Gegenwart eines Katalysators, beispielsweise einer starken Base, z.B. von Natrium- oder Kaliumhydroxid, oder einer starken Säure, wie einer

Mineralsäure, z.B. von Salzsäure, Schwefelsäure oder Phosphorsäure, oder einer organischen Sulfonsäure, z.B. von p-Toluolsulfonsäure, oder einer Lewissäure, z.B. von Bortrifluorid-Aetherat, zu einer anderen veresterten Carboxylgruppe R umgeestert werden.

Eine amidierte Carboxylgruppe R kann in üblicher Weise, z.B. durch Hydrolyse in Gegenwart eines Katalysators, beispielsweise einer starken Base, wie eines Alkalimetall- oder Erdalkalimetallhydroxides oder -carbonates, z.B. von Natrium- oder Kaliumhydroxid oder -carbonat, oder einer starken Säure, wie einer Mineralsäure, z.B. von Salzsäure, Schwefelsäure oder Phosphorsäure in die freie Carboxylgruppe R umgewandelt werden.

In einer erfindungsgemäss erhältlichen Verbindung kann man weiterhin gegebenenfalls veresterte oder verätherte Hydroxygruppen $R_2$ ineinander umwandeln.

So kann man beispielsweise eine freie Hydroxylgruppe $R_2$ durch Umsetzung mit einer vorzugsweise funktionell abgewandelten Carbonsäure, wie Niederalkancarbonsäure, z.B. Essigsäure, zu einer mit einer Carbonsäure veresterten Hydroxygruppe $R_1$ und/oder $R_2$ verestern oder durch Umsetzung mit einem Verätherungsmittel, z.B. mit einem Niederalkylierungsmittel, zu einer verätherten Hydroxygruppe, z.B. einer Niederalkoxygruppe, $R_1$ und/oder $R_2$, veräthern.

Eine funktionell abgewandelte Carbonsäure ist dabei beispielsweise ein Anhydrid, wie das symmetrische Anhydrid derselben, oder ein Anhydrid mit einer Halogen-, wie der Chlor- oder Bromwasserstoffsäure, ein reaktionsfähiger Ester d.h. ein Ester mit elektronenanziehenden Strukturen, z.B. ein Niederalkancarbonsäurephenyl-, -(p-

nitro)-phenyl- oder -cyanmethylester, oder ein reaktives Amid, z.B. ein N-Niederalkanoylimidazol oder -3,5-dimethylpyrazol.

Veräthernde Mittel sind beispielsweise reaktionsfähige veresterte Alkohole, wie mit einer Mineralsäure, z.B. mit Jod-, Chlor- oder Bromwasserstoff- oder Schwefelsäure, oder organischen Sulfonsäure, z.B. mit p-Toluol-, p-Brombenzol-, Benzol-, Methan-, Aethan- oder Aethensulfonsäure, oder Fluorsulfonsäure veresterte Alkohole, sowie Diazoalkane. Als veräthernde Mittel sind insbesondere Niederalkylchloride, -jodide, -bromide, z.B. Methyljodid, Diniederalkylsulfate, z.B. Dimethyl- oder Diäthylsulfat oder Methylfluorosulfonat, Niederalkylsulfonate, wie Niederalkyl-, z.B. Methyl-, p-toluol-, -p-brombenzol-, -methan- oder -äthansulfonate, sowie Diazoalkane, z.B. Diazomethan, zu nennen.

Die Umsetzungen mit, vorzugsweise funktionell abgewandelten, Säuren bzw. mit Verätherungsmitteln, z.B. den vorstehend hervorgehobenen, kann in üblicher Weise durchgeführt werden, bei der Umsetzung mit einem Diazoalkan, z.B. in einem inerten Lösungsmittel, wie einem Aether, z.B. in Tetrahydrofuran, oder bei der Verwendung von reaktionsfähigen veresterten Alkoholen beispielsweise in Gegenwart eines basischen Kondensationsmittels, wie einer anorganischen Base, z.B. von Natrium-, Kalium- oder Calciumhydroxid oder -carbonat, oder einer tertiären oder quaternären Stickstoffbase, z.B. von Pyridin, α-Picolin, Chinolin, Triäthylamin, oder Tetraäthyl- oder Benzyltriäthylammoniumhydroxid, und/ oder eines für die jeweilige Umsetzung üblichen Lösungsmittels, welches auch aus einem Ueberschuss des für die Veresterung verwendeten funktionellen Säurederivates, z.B. eines Niederalkansäureanhydrides oder -chlorides, oder für

die Verätherung beispielsweise verwendeten Niederalkylhalogenides oder -sulfates, und/oder einer als basisches Kondensationsmittel verwendeten tertiären Stickstoffbase, z.B. Triäthylamin oder Pyridin, bestehen kann, erforderlichenfalls bei erhöhter Temperatur. Empfehlenswert ist insbesondere die Methylierung mittels Methyljodid im Amylalkohol/Kaliumcarbonat bei Siedetemperatur, sowie die Acylierung mittels eines Niederalkansäureanhydrides bei 50-150° oder mittels eines Niederalkanoylchlorides in Pyridin oder Pyridin/Triäthylamin bei Temperaturen zwischen -20 und +100°C.

Umgekehrt kann man auch veräthertes oder vor allem verestertes Hydroxy $R_2$ in üblicher Weise, beispielsweise in Gegenwart eines sauren Mittels, wie einer Halogenwasserstoffsäure, z.B. von Jodwasserstoffsäure, in einem inerten Lösungsmittel, z.B. in Aethanol oder Essigsäure, in Hydroxy umwandeln.

Ferner kann man in einer erfindunggemäss erhältlichen Verbindung Acyl $R_2$ und/oder vor allem $R_1$ durch Wasserstoff ersetzen. So kann man eine Carboxygruppe $R_2$ und/oder vor allem $R_1$ in üblicher Weise, z.B. thermisch, decarboxylieren oder die Acylgruppe $R_1$ einer Carbonsäure in üblicher Weise, wie durch Einwirkung basischer Mittel, wie von Alkalien, z.B. von verdünnter Natronlauge oder vor allem Sodalösung, vorzugsweise von etwa 5%-iger Sodalösung, abspalten.

Die neuen Verbindungen können, je nach der Wahl der Ausgangsstoffe und Arbeitsweisen, in Form eines der möglichen Isomeren oder als Gemisch derselben, z.B. als Isomeren bezüglich der Orientierung von X, ferner je nach der Anzahl der asymmetrischen Kohlenstoffatome als reine op-

tische Isomere, wie Antipoden oder als Isomerengemische, wie Racemate, Diastereoisomerengemische oder Racematgemische, vorliegen.

Erhaltene Isomerengemische bezüglich der Orientierung von X, Diastereomerengemische und Racematgemische können auf Grund der physikalisch-chemischen Unterschiede der Bestandteile in bekannter Weise in die reinen Isomeren, Diastereomeren oder Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen, oder durch Umsetzen eines sauren Endstoffes mit einer mit der racemischen Säure Salze bildenden optisch aktiven Base und Trennung der auf diese Weise erhaltenen Salze, z.B. auf Grund ihrer verschiedenen Löslichkeiten, in die Diastereomeren, aus denen die Antipoden durch Einwirkung geeigneter Mittel freigesetzt werden können, zerlegen. Vorteilhaft isoliert man den wirksameren der beiden Antipoden.

Erhaltene freie Verbindungen der Formel I, z.B. solche, worin R, $R_1$ und/oder $R_2$ für Carboxy steht, können in an sich bekannter Weise in Salze überführt werden, u.a. durch Behandeln mit einer Base oder mit einem geeigneten Salz einer Carbonsäure, üblicherweise in Gegenwart eines Lösungs- oder Verdünnungsmittels.

Erhaltene Salze können in an sich bekannter Weise in die freien Verbindungen umgewandelt werden, z.B. durch Behandeln mit einem sauren Reagens, wie einer Mineralsäure.

Die Verbindungen einschliesslich ihrer Salze können auch in Form ihrer Hydrate erhalten werden oder das zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes und/oder Racetates bzw. Antipoden verwendet oder insbesondere unter den Reaktionsbedingungen bildet.

So kann man beispielsweise in einer Abwandlung des erfindungsgemässen Cyclisierungsverfahrens von einer Verbindung der Formel

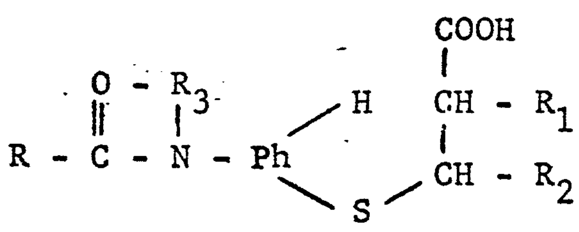

(V) ,

worin $R_1$, $R_3$, R und Ph die angegebenen Bedeutungen haben, und $R_2$ Wasserstoff oder einen gegebenenfalls substituierten, gegebenenfalls heteroanalogen Kohlenwasserstoffrest bedeutet ausgehen und diese in Gegenwart eines Anhydrides der Phorphorsäure, z.B. mit Phosphorpentoxid, Phosphorpentachlorid oder Phosphoroxychlorid, umsetzen, Dabei wird intermediär eine Verbindung der allgemeinen Formel (IV), z.B.

eine solche, in der $Y_2$ eine Gruppe der Formel $-SCR_2=CR_1-COOH$ und $Y_1$ Wasserstoff bedeutet, gebildet, die unter den Reaktionsbedingungen cyclisiert.

In analoger Weise kann man auch eine Verbindung der Formel

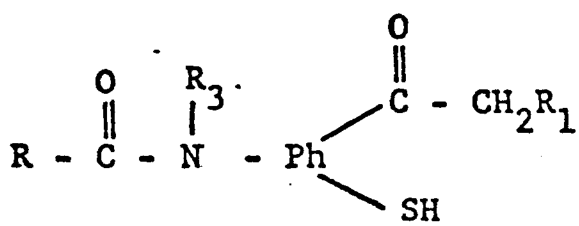

(VI) ,

mit einer in einer Anhydridform, z.B. als Halogenid, wie Chlorid, oder als symmetrisches Anhydrid, vorliegenden Säure der Formel $R_2-COOH$, wobei $R_1$ und $R_2$ unabhängig voneinander Wasserstoff oder einen gegebenenfalls substituierten, gegebenenfalls heteroanalogen Kohlenwasserstoffrest bedeuten, in Gegenwart eines basischen Kondensationsmittels, bei der Umsetzung mit einem Säurehalogenid beispielsweise eines Alkalimetallcarbonates, z.B. von Kaliumcarbonat in Aceton, und bei der Umsetzung mit einem symmetrischen Säureanhydrid beispielsweise eines Alkalimetall- oder Ammoniumcarboxylates, z.B. von Natriumacetat oder dem Natriumsalz einer der dem verwendeten Anhydrid entsprechenden Säuren, zu einer Verbindung der allgemeinen Formel (I) kondensieren, worin -SX- eine Gruppe $-S-CR_2=CR_1-CO-$ bedeutet, und $R_1$ und $R_2$ die vorstehend angegebenen Bedeutungen haben oder $R_1$ eine Gruppe $R_2CO-$ darstellt. Dabei wird intermediär eine Verbindung der allgemeinen Formel IV gebildet, in der $Y_1$ eine Gruppe $-COY_5$ der Formel $-COCH_2R_1$ und $Y_2$ eine Gruppe $-SOCY_6$ der Formel $-SOCR_2$ bedeutet, die, gegebenenfalls nach vorhergehender Acylierung der Gruppe $-COCH_2R_1$, z.B. der Formel $-COCH_3$, zu einer Gruppe $-CO-CHR_1-CO-R_2$, z.B. der Formel $-COCH_2COR_2$, erfindungsgemäss cyclisiert.

In einer anderen Abwandlung des erfindungsgemässen Cyclisierungsverfahrens kann man beispielsweise eine Verbindung der Formel

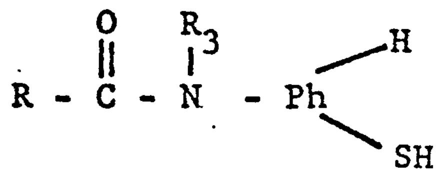

(VII),

worin R, $R_3$ und Ph die eingangs angegebenen Bedeutungen haben, in Gegenwart eines stark sauren Kondensationsmittels, wie einer Mineralsäure, z.B. von Schwefelsäure, Chlor- oder Bromwasserstoffsäure, Phophor- oder Polyphosphorsäure, oder eines aprotischen sauren Kondensationsmittels, wie eines anorganischen Säureanhydrides, z.B. von Phosphorpentachlorid oder Phosphoroxychlorid, mit einem Ester einer Säure der Formel $R_2$-CO-$CHR_1$-COOH, worin $R_1$ und $R_2$ unabhängig voneinander Wasserstoff oder einen gegebenenfalls substituierten, gegebenenfalls heteroanalogen Kohlenwasserstoffrest bedeuten, zu Verbindungen der allgemeinen Formel (I) kondensieren, in der -SX- eine Gruppe -S-$CR_2$-$CR_1$-CO- mit den vorstehenden Bedeutungen von $R_1$ und $R_2$ darstellt. Dabei wird intermediär mindestens eine Verbindung der allgemeinen Formel (IV) gebildet, z.B. eine solche, in der $Y_1$ eine Gruppe der Formel -$COCHR_1$-C(O)$R_2$- oder eine in Esterform vorliegende Gruppe der Formel -$CR_2$(OH)-$CHR_1$-COOH und $Y_2$ Mercapto bedeutet und/oder eine solche in der $Y_1$ eine in Esterform vorliegende Gruppe der Formel -$SCR_2$=$CR_1$-COOH und $Y_2$ Wasserstoff darstellt, die unter den Reaktionsbedingungen erfindungsgemäss cyclisiert.

Die Ausgangsstoffe sind bekannt oder können, sofern sie neu sind nach an sich bekannten Methoden hergestellt werden.

So kann man die Ausgangsstoffe der allgemeinen Formel (II) beispielsweise herstellen, indem man in einer Verbindung der Formel

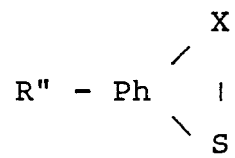

(IIa),

in der Ph und X die angegebenen Bedeutungen haben und R" Nitro oder eine von der Gruppe der Formel $RCONR_3$- verschiedene acylierte Aminogruppe bedeutet, die Nitrogruppe R" durch übliche Reduktion, z.B. katalytisch oder mit einem Metall und einer Säure, z.B. mit Eisen und Salzsäure, oder Natriumhyposulfit in wässrigem Ammoniak, in primäres Amino oder eine acylierte Aminogruppe R" durch übliche Hydrolyse, vorzugsweise in Gegenwart einer Säure, wie einer Mineralsäure, z.B. von Salz- oder Schwefelsäure, oder einer anorganischen Base, z.B. von Natron- oder Kalilauge, in eine Aminogruppe-$NHR_3$ überführt. Zunächst gebildetes primäres Amino kann leicht, z.B. mit einem Niederalkylhalogenid, zu $-NHR_3$ alkyliert werden.

Ausgangsstoffe der allgemeinen Formel (II) können ferner hergestellt werden, indem man eine Verbindung der allgemeinen Formel

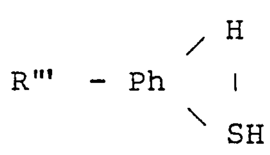

(IIb)

mit einem Ester einer Säure der Formel $R_2CICHR_1COOH$ umsetzt oder in eine Verbindung der Formel

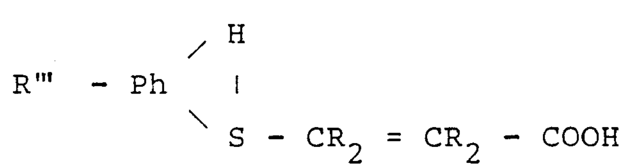

(IIc)

wobei R"' Wasserstoff oder eine Gruppe $-NHR_2$ oder R" bedeutet, Wasserstoff R"' durch übliche Nitrierung durch

Nitro ersetzt, Nitro zu Amino reduziert und erforderlichenfalls Acylamino zu Amino hydrolysiert und/oder Amino zu $-NHR_3$ alkyliert. Dabei arbeitet man in üblicher Weise, z.B. in Gegenwart eines stark sauren Kondensationsmittels, wie einer Mineralsäure, z.B. von Schwefelsäure, Chlor- oder Bromwasserstoffsäure, Phosphor- oder Polyphosphorsäure, oder vorzugsweise aprotischen sauren Kondensationsmittels, wie einem Säureanhydrid, z.B. von Phosphorpentoxid, Phosphorpentachlorid oder Phosphoroxychlorid. In analoger Weise können auch die vorstehend als Ausgangsstoffe genannten Verbindungen der Formel IIa hergestellt werden.

Die als Ausgangsstoffe genannten Verbindungen der Formel III sind grösstenteils neu. Sie weisen teilweise neben ihrer Verwendbarkeit als Ausgangsstoffe für die Herstellung von Verbindungen der Formel I weitere vorteilhafte Eigenschaften auf. So besitzen Verbindungen der Formel III, in denen R' eine gegebenenfalls verätherte Glykoloylaminogruppe bedeutet, die gleichen pharmakologischen Eigenschaften, in vergleichbarer Wirkungsstärke, wie die entsprechenden Verbindungen der Formel I.

Die Erfindung betrifft dementsprechend ebenfalls neuen Ausgangsstoffe, vor allem Verbindungen der Formel III, worin R' eine Gruppe der Formel $R_o-NHR_3-$ und $R_o$ eine gegebenenfalls verätherte Glykoloylgruppe bedeutet, Verfahren zu deren Herstellung, diese enthaltende pharmazeutische Präparate und ihre Verwendung als Pharmazeutika oder zur Herstellung von Arzneimitteln.

Verätherte Glykoloylgruppen sind beispielsweise durch einen gegebenenfalls substituierten aliphatischen oder araliphatischen Alkohol verätherte Glykoloylgruppen, wie entsprechende Niederalkoxyacetyl- oder Phenylniederalkoxyacetylgruppen. Substituenten von Niederalkoxyacetyl sind vor allem Hydroxy, Niederalkoxy und/oder Diniederalkylamino, und solche von Phenylniederalkoxyacetylgruppen, z.B. Niederalkyl, wie Methyl, Niederalkoxy, wie Methoxy, und/oder Halogen, wie Chlor. Niederalkoxy hat dabei vorzugsweise eine der eingangs angeführten Bedeutungen, Phenylniederalkoxyacetyl ist insbesondere Benzyloxy- oder 2-Phenyläthoxyacetyl. Diniederalkylaminoniederalkoxyacetyl ist vorzugsweise 2-Dimethyl- oder 2-Diäthylaminoäthoxyacetyl.

Die Erfindung betrifft dabei insbesondere solche Verbindungen der Formel III in denen Ph und $R_1$ die für die jeweils bevorzugten Verbindungsgruppen der Formel I angegebenen Bedeutungen haben, R' eine Gruppe $R_o$-$NHR_3$ und $R_o$ Niederalkoxyacetyl, vor allem mit bis zu 6 Kohlenstoffatomen, wie Methoxy- oder Aethoxyacetyl oder vorzugsweise Glykoloyl bedeutet.

Die als Ausgangsstoffe genannten Verbindungen der Formel III können nach an sich bekannten Methoden hergestellt werden, vorzugsweise, indem man eine Verbindung der Formel

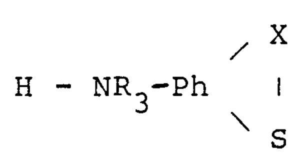

(II)

oder ein Säureadditionssalz davon mit einer entsprechenden Säure, z.B. der Formel $X_1$-OH (IIIa) bzw. $X_2$-OH (IIIb) oder einem funktionellen Derivat davon umsetzt, und gewünschtenfalls eine so erhältliche Verbindung in eine andere Verbindung der Formel III, worin R' eine Gruppe $R_o$-$NHR_3$ bedeutet, überführt.

Funktionelle Derivate von Säuren der Formel IIIa

bzw. IIIb sind vor allem veresterte, amidierte oder anhydridisierte Carboxygruppen, wie Niederalkoxycarbonyl, gegebenenfalls substituiertes Carbamyl, z.B. Carbamyl oder Imidazolyl-1-carbonyl, oder Halogencarbonyl, z.B. Chlor- oder Bromcarbonyl, oder eine Gruppe der Formel $-CON_3$ oder $CON_2^{\oplus}$ $Hal^{\ominus}$ enthaltende Säurederivate. Als Beispiele für Säuren der Formel IIIa bzw. IIIb und deren funktionelle Derivate seien insbesondere genannt: Als funktionelle Derivate von Säuren der Formel IIIa Oxalylhalogenide wie Oxalylchlorid oder Oxalylbromid, Triniederalkoxy- und Dihalogenniederalkoxyessigsäureniederalkylester, wie Oxalsäuretetraäthylester oder Dichloroxalsäurediäthylester, Oxalsäureiminodialkylester, wie Oxalsäuremono- oder -diiminodiäthylester, Oxalsäureamidine, wie N-Niederalkyloxalsäureesteramidine, Oxalsäuredithioniederalkyl-, wie -dimethylester, Cyanoformylchlorid oder Cyanogen und als Säuren der Formel IIIb und deren funktionelle Derivate Glykolsäure und ihre Niederalkylester bzw. das entsprechende Lactid, Mono- oder Diniederalkoxyessigsäureniederalkyl-, wie -äthylester, z.B. Aethoxy- oder Diäthoxyessigsäureäthylester, Halogenacetanhydride, wie Chloracetanhydrid oder Chloracetylchlorid und Weinsäure, bzw. 2,3-Diacetoxybernsteinsäureanhydrid, ferner Cinnamoylchlorid, Acetylchlorid und Glycin.

Die Umsetzung von Verbindungen der Formel II mit Säuren der Formel IIIa bzw. IIIb und deren Derivate kann in üblicher Weise erfolgen, beispielsweise in Gegenwart eines wasserbindenden Mittels, wie eines Säureanhydrides, z.B. von Phosphorpentoxid, oder von Dicyclohexylcarbodiimid, oder eines, z.B. sauren oder basischen, Kondensationsmittels, wie einer Mineralsäure, z.B. von Chlorwasserstoffsäure, oder eines Alkalimetallhydroxydes oder -carbonates, z.B. von Natrium- oder Kaliumhydroxyd, oder einer organischen Stickstoffbase, z.B. von Triäthylamin oder Pyridin. Bei der Umsetzung mit einem Säureanhydrid, wie Säurechlorid, verwendet man vorzugsweise eine organische Stickstoffbase als Kondensationsmittel. Die Umsetzung mit Carbonsäuren führt man vorzugsweise in Gegenwart eines wasserbindenden Mittels durch. Erforderlichenfalls arbeitet man jeweils in einem inerten Lösungsmittel, bei normaler Temperatur oder unter Kühlen oder Erwärmen, z.B. im Temperaturbereich von etwa 0°C bis etwa 100 °C, in einem geschlossenen Gefäss und/oder unter Inertgas, z.B. Stickstoff.

Analog kann man Verbindungen der Formel III, in denen R' eine Gruppe R-CH=N- bedeutet, durch Kondensation mit der gegebenenfalls veresterten oder amidierten Glyoxylsäure herstellen.

Verbindungen der Formel III, in denen R' für eine Gruppe $-NHR_3-X_2$ und $X_2$ für Glyoxyloyl steht, können ferner hergestellt werden, indem man eine entsprechende Halogen-, wie Bromacetylverbindung mit Hexamethylentetramin, vorzugsweise in einem wässrigen Alkohol erhitzt oder mit Silbertetrafluoroborat in Dimethylsulfoxid oxydiert. Analog kann man auch eine Chloracetylverbindung mit Kaliumdichromat in Hexamethylphosphorsäuretriamid in Gegenwart von Dicyclohexyl-18-crown-6-äther oxydieren. Verbindungen der Formel III, in denen R' eine Gruppe $X_2-NHR_3-$ und $X_2$ eine Iminoacetylgruppe, z.B. gegebenenfalls substituiertes Benzyliminoacetyl, bedeutet, können ausgehend von den entsprechenden Glycylverbindungen hergestellt werden, indem man diese mit der entsprechenden Carbonylverbindung, z.B. mit Benzaldehyd, umsetzt und das so erhältliche Zwischenprodukt, z.B. eine Benzylidenglycylverbindung, vorzugsweise unter den Reaktionsbedingungen, umlagert.

Als funktionell abgewandelte Carboxygruppe eine Iminoäthergruppierung aufweisende funktionell abgewandelte Oxalogruppen können ausgehend von der entsprechenden Cyanocarbonylverbindung durch Umsetzung mit dem entsprechenden Alkohol, z.B. Niederalkan(di)ol oder Aminoniederalkanol, hergestellt werden.

Die erfindungsgemässen Verbindungen der Formel III, worin R' eine Gruppe der Formel $R_o$-$NHR_3$- und $R_o$ eine gegebenenfalls verätherte Glykoloylgruppe bedeutet, können ferner hergestellt werden, indem man in einer Verbindung der Formel III, worin R' einen in die Gruppe $R_oNHR_3$- überführbaren Rest bedeutet, den Rest R' in die gewünschte Gruppe $R_o$-$NHR_3$- überführt und gewünschtenfalls eine so erhältliche Verbindung in eine andere Verbindung der Formel III, worin R' eine Gruppe $R_oNHR_3$- bedeutet, überführt.

In eine Gruppe $R_o$-$NHR_3$- überführbare Reste sind beispielsweise solche der Formel $X_1'$-$NHR_3$-, worin $X_1'$ eine veresterte Glykoloylgruppe, beispielsweise eine mit einer Mineralsäure, z.B. mit einer Halogenwasserstoffsäure veresterte Glykoloylgruppe, wie Chloracetyl oder Bromacetyl, bedeutet. Derartige Gruppen $X_1'$ können hydrolytisch, z.B. in Gegenwart eines basischen Hydrolysemittels, wie Natronlauge, zur Glykoloylgruppe oder durch Umsetzung mit einem Salz, wie Alkalimetallsalz, z.B. Natriumsalz, eines entsprechenden Alkohols, in veräthertes Glykoloylgruppen überführt werden.

Weitere in Gruppen der Formel $R_o$-$NHR_3$- überführbare Reste R' sind solche der Formel $X_2'$-$NHR_3$-, worin $X_2'$ einen reduktiv in die Glykoloylgruppe überführbaren Rest, wie die gegebenenfalls hydratisierte Glyoxylgruppe, bedeutet. Diese kann auch unter den Reduktionsbedingungen, z.B. aus der gegebenenfalls in Salzform wie in Natriumsalzform, oder in einer Anhydrid- oder Esterform, wie als Halogen-, z.B. Chlor- oder Bromoxalyl oder gemischtes Anhydrid mit Diphenylphosphorsäure oder in Niederalkyl- z.B. Methyl- oder Isopropylesterform, vorliegenden Oxalogruppe gebildet werden. Die Reduktion derartiger Gruppen erfolgt in üblicher Weise. Ausgehend von Halogenoxalyl verwendet man vorzugsweise, z.B. durch Palladium auf einem Träger, wie Bariumsulfat, erforderlichenfalls in Gegenwart eines schwefelhaltigen Co-katalysators, wie Thioharnstoff, katalytisch aktivierten Wasserstoff. Anhydride mit Diphenylphosphorsäure werden vorteilhaft mit einem Ueberschuss von Natriumboranat reduziert. In Salzform vorliegende Oxalogruppen werden vorteilhaft mit einem Boren, wie Diboran oder einem Boran-Aetherkomplex, z.B. mit Boran in Tetrahydrofuran reduziert, während man in einer Esterform vorliegende Oxalogruppen vorteilhaft mit Natriumanilinoborhydrid, erhältlich durch Umsetzung von Natriumborhydrid und Acetanilid in Pyridin, reduziert.

Die erfindungsgemässen Verbindungen der Formel III, worin R' eine Gruppe $R_o$-$NHR_3$- bedeutet, können ferner in strenger Analogie zu den vorstehend beschriebenen, zu Verbindungen der Formel I führenden Cyclisierungsverfahren hergestellt werden, indem man von einem Ausgangsstoff der Formeln IV, V, VI oder VII ausgeht, worin der Rest Ph statt der Gruppe R-C(=O)-$NR_3$- die gewünschte Gruppe $R_o$-$NHR_3$- trägt. Die Ausgangsstoffe dafür können ebenfalls in Analogie zu den nachstehend beschriebenen Bildungsweisen für die zur Herstellung von Verbindungen der Formel I zu verwendenden Ausgangsstoffe hergestellt werden.

Eine so erhältliche erfindungsgemässe Verbindung der Formel III kann man in eine andere erfindungsgemässe

Verbindung der Formel III umwandeln.

So kann man z.B. die gegenseitigen Umwandlungen von gegebenenfalls veresterten oder veräthherten Hydroxygruppen $R_2$ und die Trennung von Isomeren bezüglich der Orientierung von X auf die erfindungsgemässen Verbindungen übertragen.

Ferner kann man Glykoloylgruppen $R_o$ durch Umsetzung mit einem veräthernden Mittel veräthert werden, z.B. durch Ueberführung in ein Alkalimetall-, wie Natriumsalz, und Umsetzung mit einem reaktiven Derivat des betreffenden Alkohols, wie einem Niederalkylhalogenid, z.B. -bromid, oder Diniederalkylsulfat.

Die Ausgangsstoffe der allgemeinen Formel IV können beispielsweise hergestellt werden, indem man in einer Verbindung der Formel

$$R'' - Ph \begin{matrix} \diagup Y_2 \\ \diagdown Y_1 \end{matrix} \qquad (IV'),$$

worin R" von $RCONR_3$- verschiedenes Acylamino, Nitro oder Amino ist und R, Ph, $Y_1$ und $Y_2$ die angegebenen Bedeutungen haben, Acylamino R" durch übliche Hydrolyse in $-NHR_3$ oder Nitro R" durch übliche Nitroreduktion in Amino überführt, gegebenenfalls niederalkyliert und $-NHR_3$ in üblicher Weise, z.B. durch Umsetzung mit einer Verbindung der Formel R-CO-Hal, worin R die angegebene Bedeutung hat und Hal Chlor oder Brom ist, vorzugsweise in Gegenwart eines basischen Kondensationsmittels, wie einer tertiären organischen Stickstoffbase, z.B. von Pyridin oder Triäthylamin, zu der gewünschten Gruppe der Formel RCO- acyliert. Nitroverbindungen (IV') reduziert man z.B. mit Natriumdithionit oder mit Eisen und Salzsäure bzw. mit Zink und Essigsäure. Entsprechende, einen von der Gruppe der Formel $RCONR_3$ verschiedenen Acylaminorest aufweisenden Acylaminoverbindungen werden z.B. wie in Gegenwart eines sauren oder basischen Mittels, z.B. von Salz- oder Schwefelsäure oder von Natron- oder Kalilauge, hydrolysiert.

Ausgangsstoffe der Formel (IV), in denen $Y_1$ eine Gruppe $-CO-CR_1=CR_2Y_4$ der Formel $-COCHR_1-C(O)R_2-$ bedeutet, in der $R_2$ Wasserstoff oder einen gegebenenfalls substituierten, gegebenenfalls heteroanalogen Kohlenwasserstoffrest darstellt, und $Y_2$ für gegebenenfalls veräthertes Mercapto steht, kann man ferner herstellen, indem man Verbindungen der Formeln

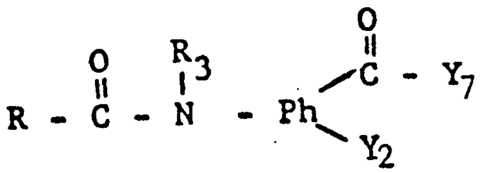 (IVb) und $R_2CO - Y_8$ (IVc)

in denen einer der Reste $Y_7$ und $Y_8$ eine Gruppe $-CH_2R_1$ und der andere veräthertes Mercapto, wie Niederalkylthio, z.B. Methyl- oder Aethylthio, bedeutet und R, $R_1$, $R_3$ und Ph die eingangs und $Y_2$ und $R_2$ die vorstehend angegebenen Bedeutungen haben, einer üblichen Esterkondensation, z.B. der Umsetzung in Gegenwart eines stark basischen Kondensationsmittels, wie eines Alkalimetalles oder des Hydrides, eines Amides oder Alkoholates eines Alkalimetalles, z.B. von Natrium, Natriumhydrid, Diisopropylaminlithium oder Natriummethylat, unterwirft und gewünschtenfalls eine ausgehend von einer Verbindung, in der $R_1$ Wasserstoff ist, gebildete Gruppe der Formel $-COCH_2C(O)R_2-$ in üblicher Weise, z.B. durch Ueberführung in ein α-Metallsalz, vorzugsweise durch Umsetzung mit einem der genannten stark basischen Kondensationsmittel, z.B. mit Diisopropylaminlithium, und anschliessende Umsetzung mit einem Halogenid, z.B. Chlorid oder Bromid, des gewünschten gegebenenfalls substituierten,

gegebenenfalls heteroanalogen Kohlenwasserstoffes oder der dem gewünschten Acylrest $R_1$ entsprechenden Säure, α-substituiert.

Ausgangsstoffe der allgemeinen Formel (IV), in denen $Y_1$ eine gegebenenfalls in Esterform vorliegende Gruppe der Formel $-SCR_2=CR_1-COOH$ und $R_2$ Wasserstoff oder einen gegebenenfalls substituierten, gegebenenfalls heteroanalogen Kohlenwasserstoffrest darstellt, und $Y_2$ für Wasserstoff steht, kann man ferner herstellen, indem man eine Verbindung der Formel IVd und eine in Esterform, z.B. als Methyl- oder Aethylester, vorliegende Verbindung der Formel IVe

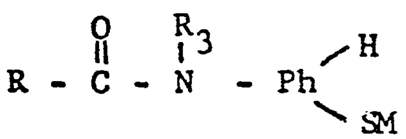 (IVd) und $Y_9 - CR_2 = Y_{10}-COOH$ (IVe)

worin entweder $Y_9$ Halogen, z.B. Chlor oder Brom, und $Y_{10}$ einen Rest $R_1$ bedeutet oder $Y_9$ und $Y_{10}$ gemeinsam eine zusätzliche Bindung darstellen, $R_1$, R, $R_3$ und Ph die eingangs und $R_2$ die vorstehend angegebenen Bedeutungen haben und M ein Alkalimetall, z.B. Natrium, bedeutet, in üblicher Weise kondensiert.

Ausgangsstoffe der allgemeinen Formel (IV), in denen $Y_2$ gegebenenfalls veräthertes oder mit einer Carbonsäure verestertes Mercapto und $Y_1$ eine Gruppe der Formel $-CR_2=CR_1-Y_3$ bedeutet, worin $R_2$ Wasserstoff oder einen gegebenenfalls substituierten, gegebenenfalls heteroanalogen Kohlenwasserstoffrest darstellt, kann man ferner herstellen, indem man eine Verbindung der Formel IVf und eine, vorzugsweise in Esterform vorliegende, Verbindung der Formel IVg

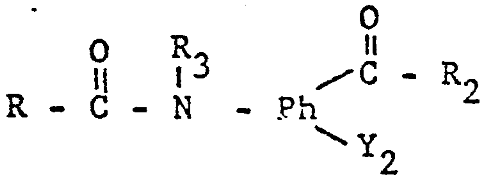 (IVf) und $Y_{11} - CHR_1 - Y_3$(IVg)

worin $R_1$, R, $R_3$ und Ph die eingangs und $R_2$ die vorstehend angegebenen Bedeutungen haben und $Y_{11}$ Wasserstoff oder Halogen, z.B. Brom oder Chlor, darstellt, in üblicher Weise, bei der Umsetzung mit α-Halogenestern, vorzugsweise in Gegenwart von Zink oder bei der Umsetzung mit Estern mittels Kaliumcarbonat und Aceton, kondensiert und in dem erhaltenen Kondensationsprodukt gewünschtenfalls veräthertes Hydroxy $Y_2$, in üblicher Weise, z.B. mittels Jodwasserstoffsäure, in Hydroxy überführt.

Die, z.B. vorstehend beschriebenen, zu Ausgangsstoffen der allgemeinen Formel (IV) führenden Aufbaureaktionen können jedoch auch in abgeänderter Reihenfolge durchgeführt werden. So kann man beispielsweise anstelle der genannten Ausgangsverbindungen IVb, IVd oder IVf Verbindungen der Formeln IVh, IVi oder IVj

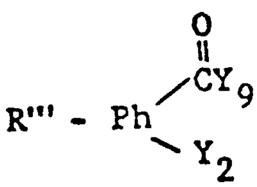 (IVh), 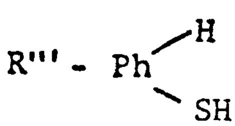 (IVi), 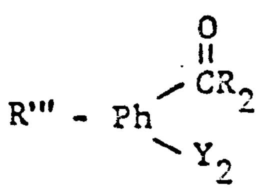 (IVj)

worin R"' Wasserstoff, Nitro, eine Gruppe $-NHR_3$ oder von der Gruppe $RCONR_3$- verschiedenes acyliertes Amino bedeutet, in der angegebenen Weise kondensieren und in der erhaltenen Verbindung der Formel (IIIa) die Gruppe R"' wie angegeben in die gewünschte Gruppe RCO- überführen.

Beim Verfahren der vorliegenden Erfindung werden vorzugsweise solche Ausgangsstoffe verwendet, welche zu den eingangs als besonders wertvoll geschilderten Verbindungen führen.

Die vorliegende Erfindung betrifft ebenfalls pharmazeutische Präparate, welche eine der erfindungsgemässen Verbindungen der Formel I bzw. III oder ein pharmazeutisch

verwendbares Salz davon enthalten. Bei den erfindungsgemässen pharmazeutischen Präparaten handelt es sich um solche, die zur topischen und lokalen sowie enteralen, wie oralen oder rektalen, sowie parenteralen Verabreichung an und zur Inhalation durch Warmblüter, bestimmt sind und den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab.

Die neuen pharmazeutischen Präparate enthalten z.B. von etwa 10% bis etwa 95%, vorzugsweise von etwa 20% bis etwa 90% des Wirkstoffs. Erfindungsgemässe pharmazeutische Präparate sind z.B. solche in Aerosol- oder Sprayform oder in Dosiseinheitsformen, wie Dragées, Tabletten, Kapseln oder Suppositorien, ferner Ampullen.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister z.B. Mais-, Weizen-, Reis- oder

Kartoffelstärkekleister, Gelatine, Tragakanth, Methylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere, oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natrium-carboxymethylcellulose, Sorbit und/oder Dextran und gegebenenfalls auch Stabilisatoren enthalten.

Inhalationspräparate für die Behandlung der Atemwege durch nasale oder buccale Verabreichung sind z.B. Aerosole oder Sprays, welche den pharmakologischen Wirkstoff in Form eines Puders oder in Form von Tropfen einer Lösung oder Suspension verteilen können. Präparate mit Puder-verteilenden Eigenschaften enthalten ausser dem Wirkstoff üblicherweise ein flüssiges Treibgas mit einem Siedepunkt unter der Raumtemperatur, sowie, wenn erwünscht, Trägerstoffe, wie flüssige oder feste nicht-ionische oder anionische oberflächenaktive Mittel und/oder Verdünnungsmittel. Präparate, in welchen der pharmakologische Wirkstoff in Lösung vorliegt, enthalten ausser diesem ein geeignetes Treibmittel, ferner, falls notwendig, ein zusätzliches Lösungsmittel und/oder einen Stabilisator. Anstelle des Treibgases kann auch Druckluft verwendet werden, wobei diese mittels einer geeigneten Verdichtungs- und Entspannungsvorrichtung nach Bedarf erzeugt werden kann.

Pharmazeutische Präparate für topische und lokale Verwendung sind z.B. für die Behandlung der Haut Lotionen und Cremen, die eine flüssige oder semifeste Oel-in-Wasser- oder Wasser-in-Oel-Emulsion enthalten, und Salben (wobei solche vorzugsweise ein Konservierungsmittel enthalten), für die Behandlung der Augen Augentropfen, welche die aktive Verbindung in wässriger oder öliger Lösung enthalten und Augensalben, die vorzugsweise in steriler Form hergestellt werden, für die Behandlung der Nase Puder, Aerosole und Sprays (ähnlich den oben beschriebenen für die Behandlung der Atemwege), sowie grobe Puder, die durch schnelles Inhalieren durch die Nasenlöcher verabreicht werden, und Nasentropfen, welche die aktive Verbindung in wässriger oder öliger Lösung enthalten, oder für die lokale Behandlung des Mundes Lutschbonbons, welche die aktive Verbindung in einer im allgemeinen aus Zucker und Gummiarabikum oder Tragakanth gebildeten Masse enthalten, welcher Geschmacksstoffe beigegeben sein können, sowie Pastillen, die den Aktivstoff in einer inerten Masse, z.B. aus Gelatine und Glycerin oder Zucker und Gummiarabikum, enthalten.

Die Erfindung betrifft ebenfalls die Verwendung der neuen Verbindungen der Formel (I) und ihrer Salze als pharmakologisch aktive Verbindungen, insbesondere als Antiallergika, vorzugsweise in der Form von pharmazeutischen Präparaten. Die tägliche Dosis, die einem Warmblüter von

etwa 70 kg verabreicht wird, beträgt von etwa 200 mg bis etwa 1200 mg.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Beispiel 1

Zu einer Lösung von 8,4 g 2-Methyl-7-amino-4-oxo-4H-1 -benzothiopyran in 80 ml Dimethylformamid und 5,6 g Triäthylamin wird unter Rühren eine Lösung von 6,8 g Oxalsäuremonomethylesterchlorid in 20 ml Dimethylformamid zugetropft. Die Zutropfgeschwindigkeit wird so einreguliert, dass die Innentemperatur 35° nicht übersteigt. Man rührt 2 Stunden bei Raumtemperatur nach giesst in 1200 ml Eiswasser und nutscht den ausgefallenen Niederschlag ab. Dieser wird getrocknet und 2-mal aus einem Gemisch von je 200 ml Chloroform und 100 ml Methanol umkristallisiert. Man erhält das 2-Methyl-7-methoxyoxalylamino-4-oxo-4H-1-benzothiopyran vom Fp. 224° (Zers.).

Das Ausgangsmaterial kann folgendermasser hergestellt werden:

Zu einer Lösung von 25 g 3-Aminothiophenol in 150 ml Essigsäureäthylester von 50° werden unter leichtem Rückfluss 12 ml Chlorameisensäureäthylester zugetropft. Man fügt 50 ml Essigsäureäthylester hinzu, lässt 5 Minuten am Rückfluss sieden, kühlt auf 40° ab und nutscht das Reaktionsgemisch ab. Das Filtrat wird zur Trockne eingedampft und der ölige Eindampfrückstand an Kieselgel mit Toluol als Laufmittel chromatographiert. Durch Eindampfen des Eluats erhält man 3-Aethoxycarbonylaminothiophenol. 10 g desselben werden mit 8 g Acetessigsäureäthylester vermischt und zu 153 g Polyphosphorsäure von 90° zugetropft. Man erwärmt 2 Stunden auf 95° Innentemperatur, giesst das heisse Reaktionsgemisch auf 2 Liter Eiswasser, rührt 30 Minuten und nutscht den ausgefallenen Niederschlag ab. Man erhält so das 2-Methyl-7-aethoxycarbonylamino-4-oxo-4H-1-benzothiopyran, welches nach Umkristallisieren aus 70 ml Aethanol bei 216-218° schmilzt. 15,6 g desselben werden mit 28,7 ml Schwefelsäure und 52 ml Essigsäure 5 Stunden zum Rückfluss erhitzt. Das Reaktionsgemisch wird auf 500 ml Eiswasser ge-gossen und mit Natronlauge neutralisiert. Man erhält das 2-Methyl-7-amino-4-oxo-4H-1-benzothiopyran vom Fp. 165-169°, welches über das Hydrochlorid gereinigt werden kann und dann bei 172-175° schmilzt.

Beispiel 2

6,6 g 2-Methyl-7-methoxyoxalylamino-4-oxo-4H-1-benzothiopyran werden in 240 ml 0,1 n Natronlauge suspendiert. Man lässt 2 Stunden bei 40° rühren, filtriert, säuert mit 2n Salzsäure an, sammelt das ausgeschiedene Rohprodukt und kocht dieses zunächst mit Dimethylformamid und anschliessend mit Aethanol aus. Man erhält das 2-Methyl-7-oxaloamino-4-oxo-4H-1-benzothiopyran vom Fp. 250° (Zers.).

Beispiel 3

Zu einer Lösung von 13,3 g 2-Methyl-6-amino-4-oxo-4H-1-benzothiopyran in 8,9 g Triäthylamin und 360 ml Dimethylformamid tropft man eine Lösung von 10,7 g Oxalsäuremonomethylesterchlorid in 24 ml Dimethylformamid hinzu. Das Reaktionsgemisch wird 2 Stunden gerührt, in 1200 ml Eiswasser eingegossen, der ausgefallene Niederschlag abgenutscht, getrocknet und aus Dimethylformamid/Methanol umkristallisiert. Man erhält 2-Methyl-6-methoxyoxalylamino-4-oxo-4H-1-benzothiopyran vom Fp. 260-262°.

Das Ausgangsmaterial kann in analoger Weise wie in Beispiel 1 beschrieben hergestellt werden, z.B. durch Umsetzung von 50 g 4-Aminothiophenol in 300 ml Essigsäureäthylester mit 24 ml Chlorameisensäureäthylester zur entsprechenden Aethoxycarbonylverbindung vom Fp. 60-61° (aus Aether/Hexan, 70 ml), Umsetzung desselben mit 8 g Acetessigsäureäthylester und 153 g Polyphosphorsäure zum 2-Methyl-6-Aethoxycarbonylamino-4-oxo-4H-1-benzothiopyran

vom Fp. 219-220° (aus Aethanol) und Umsetzung desselben mit 12,7 ml Schwefelsäure und 23 ml Essigsäure zum 2-Methyl-6-amino-4-oxo-4H-1-benzothiopyran vom Fp. 265° (aus Aethanol).

Beispiel 4

In analoger Weise wie in Beispiel 2 beschrieben erhält man durch Umsetzung von 13 g 2-Methyl-6-methoxyoxalylamino-4-oxo-4H-1-benzothiopyran mit 470 ml 0,1n Natronlauge das 2-Methyl-6-oxaloamino-4-oxo-4H-1-benzothiopyran vom Fp. 238-240° (aus Dimethylformamid)

Beispiel 5

In analoger Weise wie in den Beispielen 1-4 beschrieben kann man ferner herstellen:

2,3-Dimethyl-7-methoxyoxalylamino-4-oxo-4H-1-benzothiopyran,
2,3-Dimethyl-7-oxaloamino-4-oxo-4H-1-benzothiopyran,
2-Phenyl-7-methoxyoxalylamino-4-oxo-4H-1-benzothiopyran und
2-Phenyl-6-oxaloamino-4-oxo-4H-1-benzothiopyran.

Beispiel 6

Tabletten, enthaltend 0,1 g 2-Methyl-7-oxaloamino-4-oxo-4H-1-benzothiopyran, werden wie folgt hergestellt:

Zusammensetzung (für 1000 Tabletten):

| | |
|---|---|
| 2-Methyl-7-oxaloamino-4-oxo-4H-1-benzothiopyran | 100 g |
| Lactose | 50 g |
| Weizenstärke | 73 g |
| Kolloidale Kieselsäure | 13 g |
| Magnesiumstearat | 2 g |
| Talk | 12 g |
| Wasser | q.s. |

Das 2-Methyl-7-oxaloamino-4-oxo-4H-1-benzothiopyran wird mit einem Teil der Weizenstärke, mit der Lactose und der kolliodalen Kieselsäure vermischt und das Gemisch durch ein Sieb getrieben. Ein weiterer Teil der Weizenstärke wird mit der fünffachen Menge Wasser auf dem Wasserbad verkleistert und die obige Pulvermischung mit diesem Kleister angeknetet, bis eine schwach plastische Masse entstanden ist. Die plastische Masse wird durch ein Sieb von etwa 3 mm Maschenweite gedrückt, getrocknet und das trockene Granulat nochmals durch ein Sieb getrieben. Darauf werden die restliche Weizenstärke, der Talk und das Magnesiumstearat zugemischt und die erhaltene Mischung zu Tabletten von 0,25 g (mit Bruchkerbe) verpresst.

In analoger Weise können auch Tabletten enthaltend jeweils 100 mg einer der in den Beispielen 1 und 2 genannten Verbindungen der allgemeinen Formel I hergestellt werden.

Beispiel 7

Eine zur Inhalation geeignete, etwa 2%-ige wässrige Lösung eines in freier Form oder in Form des Natriumsalzes wasserlöslichen erfindungsgemässen Wirkstoffes kann z.B. in folgender Zusammensetzung hergestellt werden:

Zusammensetzung:

| | |
|---|---|
| Wirkstoff, z.B. 2-Methyl-7-oxaloamino-4-oxo-4H-1-benzothiopyran | 2000 mg |
| Stabilisator, z.B. Aethylendiamintetraessigsäure-dinatriumsalz | 10 mg |
| Konservierungsmittel, z.B. Benzalkoniumchlorid, | 10 mg |
| Wasser, frisch destilliert, | ad 100 mg |

Herstellung

Der Wirkstoff wird unter Zusatz der äquimolekularen Menge 2n-Natronlauge in frisch destilliertem Wasser gelöst. Dann wird der Stabilisator und das Konservierungsmittel hinzugegeben. Nach vollständiger Auflösung aller Komponenten wird die erhaltene Lösung auf 100 ml aufgefüllt, in Fläschchen abgefüllt und diese gasdicht verschlossen.

In analoger Weise können auch 2%-ige Inhalationslösungen enthaltend eine Zielverbindung eines der Beispiele 1, 2 und 4 als Wirkstoff hergestellt werden.

Beispiel 8

Zur Insufflation geeignete, etwa 25 mg eines erfindungsgemässen Wirkstoffes enthaltende Kapseln können z.B. in folgender Zusammensetzung hergestellt werden:

Zusammensetzung

| | |
|---|---|
| Wirkstoff, z.B. 2-Methyl-7-methoxyoxalyl-amino-4-oxo-4H-1-benzothiopyran | 25 g |
| Lactose, feinst gemahlen | 25 g |

Herstellung

Der Wirkstoff und die Lactose werden innig vermischt, Das erhaltene Pulver wird sodann gesiebt und in Portionen zu je 50 mg in 1000 Gelatinekapseln abgefüllt.

In analoger Weise können auch Insufflationskapseln enthaltend jeweils eine Zielverbindung gemäss einem der Beispiele 2 bis 5 hergestellt werden.

Patentansprüche

1. Neue Benzothiopyranderivate der allgemeinen Formel

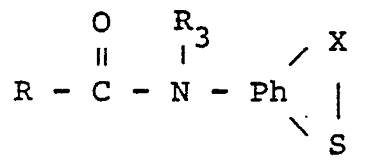

(I)

worin R gegebenenfalls verestertes oder amidiertes Carboxy oder gegebenenfalls veräthertes Hydroxymethyl bedeutet, Ph die Gruppe R-CO-$NR_3$- enthaltendes, gegebenenfalls substituiertes 1,2-Phenylen bedeutet, X eine Gruppe der Formel -CO-$CR_1$=$CR_2$- bedeutet, in der $R_1$ und $R_2$ unabhängig voneinander Wasserstoff, Acyl oder einen gegebenenfalls substituierten, gegebenenfalls heteroanalogen Kohlenwasserstoffrest oder gemeinsam 3- bis 5-gliedriges Niederalkylen bedeuten und $R_2$ auch gegebenenfalls veräthertes oder mit einer organischen Carbonsäure verestertes Hydroxy sein kann, und $R_3$ Wasserstoff oder Niederalkyl bedeutet, in freier Form oder in Salzform.

2. Verbindungen gemäss Anspruch 1, worin R Carboxy, mit einem gegebenenfalls durch gegebenenfalls substituiertes Phenyl substituierten Niederalkanol verestertes Carboxy bzw. veräthertes Hydroxymethyl, mit einem gegebenenfalls substituierten Phenol verestertes Carboxy oder gegebenenfalls durch Niederalkyl, gegebenenfalls substituiertes Phenylniederalkyl oder gegebenenfalls substituiertes Phenyl mono- oder durch Niederalkyl oder gegebenenfalls heteroanaloges Niederalkylen disubstituiertes Carbamyl bedeutet, Ph die Gruppe R-CO-$NR_3$- enthaltendes, gegebenenfalls substituiertes 1,2-Phenylen bedeutet, X eine Gruppe -CO-$CR_1$=$CR_2$- bedeutet, in der $R_1$ und $R_2$ unabhängig voneinander Wasserstoff, Niederalkanoyl, gegebenenfalls mit einem Niederalkanol verestertes Carboxy, gegebenenfalls durch Phenyl, das seinerseits substituiert sein kann, substituiertes Niederalkyl oder gegebenenfalls substituiertes Phenyl oder 5- bis 6-gliedriges, ein Stickstoff-, Sauerstoff- oder Schwefelatom aufweisendes Heteroaryl oder gemeinsam Tri-, Tetra- oder Pentamethylen darstellen und $R_2$ auch gegebenenfalls mit einem Niederalkanol veräthertes oder mit einer Niederalkancarbonsäure verestertes Hydroxy bedeuten kann, und $R_3$ Wasserstoff oder Niederalkyl bedeutet, wobei als Substituenten von Phenyl, Phenol, 1,2-Phenylen Ph und Heteroaryl, Niederalkyl, Niederalkoxy, Halogen, Hydroxy und Trifluormethyl in Betracht kommen, in freier Form oder in Salzform.

3. Verbindungen einerseits der allgemeinen Formel Ia

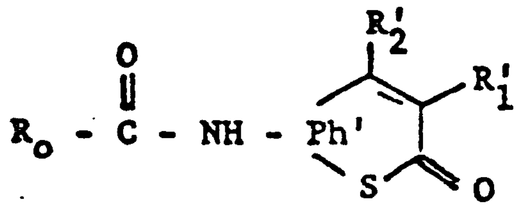

(Ia)

andererseits Verbindungen der allgemeinen Formel Ib

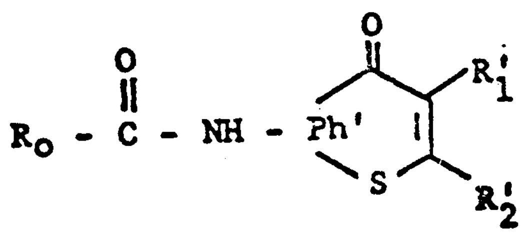

(Ib)

worin jeweils $R_o$ Carboxy, Niederalkoxycarbonyl, Hydroxymethyl, Niederalkoxymethyl, im Phenylteil gegebenenfalls wie nachstehend angegeben substituiertes Phenylniederalkoxycarbonyl, oder gegebenenfalls durch Niederalkyl mono- oder di-substituiertes oder durch Tetra- oder Pentamethylen bzw. 3-Oxa-, 3-Aza- oder 3-Thiapentamethylen disubstituiertes Carbamyl bedeutet, Ph' die Gruppe $R_o$-CO-NH- enthaltendes, zusätzliches gegebenenfalls wie nachstehend angegeben substituiertes 1,2-Phenylen bedeutet, $R_1'$ und $R_2'$ gemeinsam Tri-, Tetra- oder Pentamethylen bedeuten oder $R_1'$ Wasserstoff, Niederalkanoyl, Carboxy, Niederalkoxycarbonyl, Niederalkyl oder gegebenenfalls wie

nachstehend angegeben substituiertes Phenyl oder Pyridyl bedeutet und $R_2'$ eine der für $R_1'$ angegebenen Bedeutungen hat oder Hydroxy, Niederalkoxy oder Niederalkanoyloxy bedeutet, wobei als Substituenten von substituiertem Phenylniederalkoxycarbonyl R', zusätzlich substituiertem 1,2-Phenylen Ph' und von substituiertem Phenyl und Pyridyl $R_1'$ und/oder $R_2'$ Niederalkyl, Niederalkoxy, Halogen, Hydroxy und Trifluormethyl in Betracht kommen, jeweils in freier Form oder in Salzform.

4. Verbindungen der Formel

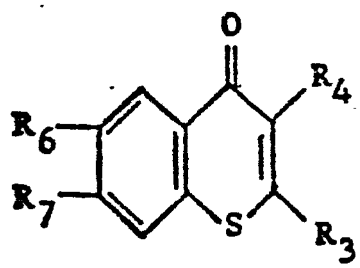

(Ic),

worin einer der Reste $R_6$ und $R_7$ eine Gruppe der Formel $R_o'$-CO-NH, in der $R_o'$ Carboxy, Hydroxymethyl oder Niederalkoxycarbonyl oder Niederalkoxymethyl mit bis zu 5 C-Atomen darstellt und der andere Wasserstoff darstellt, und $R_3$ und $R_4$ unabhängig voneinander Wasserstoff oder Niederalkyl mit bis zu 4 C-Atomen bedeuten, in freier Form oder in Salzform.

5. 2-Methyl-7-methoxyoxalylamino-4-oxo-4H-1-benzothiopyran.

6. 2-Methyl-7-oxaloamino-4-oxo-4H-1-benzothiopyran oder ein Salz davon.

7. 2-Methyl-6-methoxyoxalylamino-4-oxo-4H-1-benzothiopyran.

8. 2-Methyl-6-oxaloamino-4-oxo-4H-1-benzothiopyran oder ein Salz davon.

9. Pharmazeutische Präparate enthaltend eine Verbindung gemäss einem der Ansprüche 1 bis 8 neben üblichen pharmazeutischen Hilfs- und Trägerstoffen.

10. Verfahren zur Herstellung neuer Benzothiopyranderivate der allgemeinen Formel

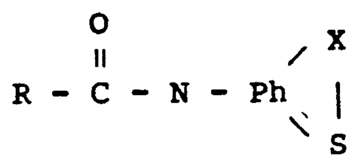 (I)

worin R gegebenenfalls verestertes oder amidiertes Carboxy oder gegebenenfalls veräthertes Hydroxymethyl bedeutet, Ph die Gruppe $R-CO-NR_3-$ enthaltendes, gegebenenfalls substituiertes 1,2-Phenylen bedeutet, X eine Gruppe der Formel $-CO-CR_1=CR_2-$ bedeutet, in der $R_1$ und $R_2$ unabhängig voneinander Wasserstoff, Acyl oder einen gegebenenfalls substituierten, gegebenenfalls heteroanalogen Kohlenwasserstoffrest oder gemeinsam 3- bis 5-gliedriges Niederalkylen bedeuten und $R_2$ auch gegebenenfalls veräthertes oder mit einer organischen Carbonsäure verestertes Hydroxy sein kann, und $R_3$ Wasserstoff oder Niederalkyl bedeutet, in freier Form oder in Salzform, dadurch gekennzeichnet, dass man eine Verbindung der allgemeinen Formel

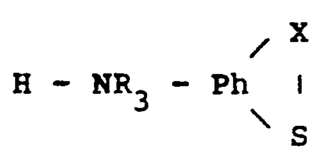 (II),

worin Ph, $R_3$ und X die angegebenen Bedeutungen haben, oder ein Salz davon mit einem Aequivalent einer gegebenenfalls funktionell abgewandelten Oxalsäure oder einem Salz davon umsetzt oder in einer Verbindung der Formel

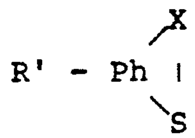

(III)

worin R' einen in die gewünschte Gruppe der Formel $RCO-NR_3-$ überführbaren Rest bedeutet, R' in die Gruppe der Formel $RCO-NR_3-$ überführt oder eine Verbindung der allgemeinen Formel

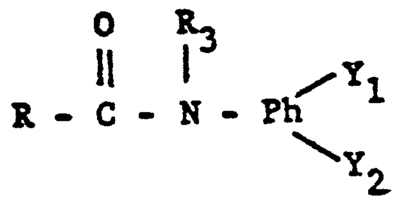

(IV) ,

worin entweder $Y_1$ eine Gruppe $-CR_2=CR_1-Y_3$ bzw. $-CO-CR_1=CR_2-Y_4$ und $Y_2$ gegebenenfalls veräthertes oder mit einer Carbonsäure verestertes Mercapto bedeutet, oder $Y_1$ Wasserstoff und $Y_2$ eine Gruppe $-S-CR_2=CR_1-Y_3$ bzw. $-S-CO-CR_1=CR_2-Y_4$ bedeutet, oder $Y_1$ eine Gruppe $-C(C)Y_5$ und $Y_2$ eine Gruppe $-S-C(O)-Y_6$ bedeutet, und worin $Y_3$ gegebenenfalls funktionell abgewandeltes Carboxy bedeutet, $Y_4$ gegebenenfalls veräthertes oder verestertes Mercapto bedeutet, wobei eine Gruppe $-CO-CR_1=CR_2-OH$ bzw. $-S-CO-CR_1=CR_2-OH$ auch in der durch die Formel $-CO-CHR_1-C(O)-R_2$ bzw. $-S-CO-CHR_1-C(O)-R_2$ dargestellten tautomeren Oxoform vorliegen kann, einer der Reste $Y_5$ und $Y_6$ eine Gruppe $-CH_2R_1$ und der andere eine Gruppe $R_2$ oder veräthertes Mercapto bedeutet, und R, Ph, $R_1$, $R_2$ und $R_3$ die angegebenen Bedeutungen haben, oder ein Salz davon intramolekular cyclisiert und in einer gegebenenfalls erhaltenen Verbindung der Formel

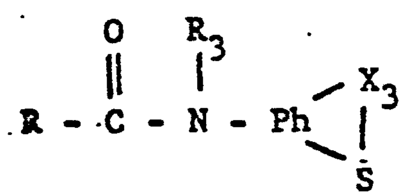

(I')

worin $X_3$ eine Gruppe $-C(=Y_o)-CR_1=CR_2-$ und $Y_o$ eine funktionell abgewandelte Oxogruppe bedeutet, $Y_o$ in die Oxogruppe überführt, erforderlichenfalls ein erhaltenes Isomerengemisch in die Komponenten auftrennt und gewünschtenfalls

eine so erhältliche Verbindung in eine andere Verbindung der Formel I umwandelt und/oder ein erhaltenes Salz in die freie Verbindung oder in ein anderes Salz oder eine erhaltene salzbildende Verbindung in ein Salz überführt.